(19) Europäisches Patentamt
European Patent Office
Office européen des brevets

(11) Publication number: **0 159 881**
**B1**

(12) **EUROPEAN PATENT SPECIFICATION**

(45) Date of publication of patent specification: **01.03.89**

(51) Int. Cl.$^4$: **C 07 H 17/08**, A 61 K 31/70

(21) Application number: **85302583.1**

(22) Date of filing: **12.04.85**

(54) **23-C-substituted mycaminosyl tylonolide.**

(30) Priority: **12.04.84 JP 73633/84**
**19.07.84 JP 150287/84**

(43) Date of publication of application:
**30.10.85 Bulletin 85/44**

(45) Publication of the grant of the patent:
**01.03.89 Bulletin 89/09**

(84) Designated Contracting States:
**DE FR GB IT**

(58) References cited:
**EP-A-0 070 170**

**BULLETIN OF THE CHEMICAL SOCIETY OF JAPAN, vol. 54, no. 12, December 1981, pages 3837-3845, JP; A. TANAKA et al.: "Syntheses of recyclized macrolide antibiotics and related derivatives from mycaminosyltylonolide"**

(73) Proprietor: **ZAIDAN HOJIN BISEIBUTSU KAGAKU KENKYU KAI**
**14-23, Kamiosaki 3-chome**
**Shinagawa-ku Tokyo (JP)**

(72) Inventor: **Umezawa, Hamao**
**23, Toyotama Kita 4-chome Nerima-ku**
**Tokyo (JP)**
Inventor: **Umezawa, Sumio**
**5-1-1-709, Shinjuku Shinjuku-ku**
**Tokyo (JP)**
Inventor: **Tsuchiya, Tsutomu**
**3-1-17-201, Edahigashi Midori-ku**
**Yokohama-shi Kanagawa (JP)**
Inventor: **Takeuchi, Tomio**
**New-Fuji-Mansion No. 5-1-11, Higashi Gotanda**
**Shinagawa-ku Tokyo (JP)**
Inventor: **Tanaka, Akihiro**
**3-17-14-304, Asahi-cho Nerima-ku**
**Tokyo (JP)**
Inventor: **Sakamoto, Shuichi**
**3-17-7-402, Sakae-cho**
**Higashimurayama-shi Tokyo (JP)**

(74) Representative: **Geering, Keith Edwin**
**REDDIE & GROSE 16 Theobalds Road**
**London WC1X 8PL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 159 881 B1

**Description**

This invention relates to antibacterial compounds which show antibacterial activity against various pathogens including gram-positive and -negative bacteria, the production of these compounds, and medical compositions containing them.

The compounds of this invention are those of the general formula (I)

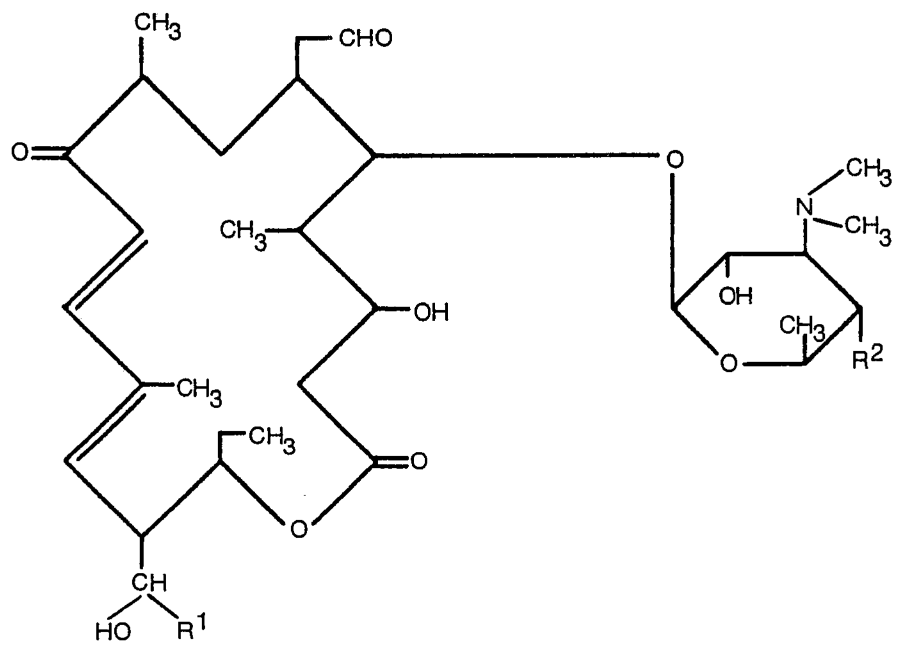

(I)

wherein $R^1$ represents a lower alkyl group, an aryl group, a lower alkenyl group or a lower alkynyl group, $R^2$ represents a hydrogen atom or a hydroxyl group. Symbols herein have the same significances throughout unless otherwise indicated.

The term "lower alkyl" in the foregoing definitions means a straight or branched carbon chain alkyl having 1 to 5 carbon atoms. Thus, examples of the "lower alkyl" are methyl, ethyl, n-propyl, iso-propyl, n-butyl, iso-butyl, sec-butyl and tert-butyl. Examples of the "aryl" in the foregoing definitions are phenyl and naphthyl. The term "lower alkenyl" in the foregoing definitions means a straight or branched carbon chain alkenyl having 2 to 6 carbon atoms and having one or two double bonds. Thus examples of the "lower alkenyl" are vinyl ($CH_2{=}CH—$), 1-propenyl ($CH_3—CH{=}CH—$), 2-propenyl ($CH_2{=}CH—CH_2—$), iso-propenyl

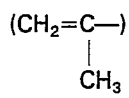

2-butenyl ($CH_3—CH{=}CH—CH_2—$) and 2-pentenyl ($CH_3—CH_3—CH{=}CH—CH_2—$). The term "lower alkinyl" in the foregoing definitions means a straight or branched carbon chain alkinyl having 2 to 6 carbon atoms and having triple bond(s). Thus examples of the "lower alkynyl" are ethynyl ($CH{\equiv}C—$), 2-propargyl ($CH{\equiv}C—CH_2—$), 2-butynyl ($CH_3—C{\equiv}C—CH_2—$) and 2-penten-4-ynyl ($CH{\equiv}C—CH{=}CH—CH_2—$).

The compounds of this invention have an asymmetric carbon atom at the 23-position, and so there are optical isomers. Thus this invention includes all of the isomers, individually and in any combination, e.g. racemic compound(s) or optically active isomer(s).

British Patent Publication 2,081,711 and European Patent Publication 70,170 disclose tylosin compounds having only mycaminose or desosamine as sugar component. These publications disclose tylosin compounds wherein the 23-position carbon atom has a substituent such as a halogen atom, a hydroxyl group, an alkanoyloxy group or

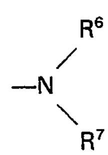

(wherein $R^6$ represents a hydrogen atom or a lower alkyl group which may be substituted by a hydroxyl group, $R^7$ represents a hydrogen atom, an alkyl group which may be substituted for example by a hydroxyl group, an aryl group, or an aralkyl group.

The compounds of this invention of the formula (I) are novel compounds having excellent antibacterial activity, and have the characteristic that the 23-position carbon atom has a substituent selected from lower alkyl, aryl, lower alkenyl, and lower alkinyl groups.

The compounds of this invention have shown antibacterial activity against various pathogens including gram-positive and -negative bacteria, and in particular the compounds have shown activity against several important gram-negative bacteria. Thus, the compounds are useful for medicaments (especially antibiotics) for the prevention or treatment of diseases caused by such bacteria.

Antibacterial medicaments containing the compounds according to this invention can be prepared by conventional methods using conventional carriers or excipients; such carriers or excipients may be organic or inorganic, solid or liquid materials which are pharmaceutically acceptable and suitable for oral or parenteral administration or for external application. They may for example by administered as capsules, tablets (such as sugar-coated tablets), ointments, suppositories, solutions, suspensions or emulsions. The appropriate dose is determined in each case considering factors such as the symptom, age and sex of the patient, but for an adult 10—1000 mg per single dose is usually administered one to four times per day.

Concerning the antimicrobial activity of the compounds of this invention of the formula (I), minimum effective inhibitory concentrations (MIC) of the compounds are shown in the following Table:

Table (MIC δ/ml) MT:mycaminosyltylonolide DT:4′deoxymycaminosyltylonolide

| Strain | Control | | Example No. | | | | | | | | |
|---|---|---|---|---|---|---|---|---|---|---|---|
| | MT | DT | 1(3) | 2(2) | 4(2) | 5(2) | 6 | 8″F″ | 10″F″ | 11″F″ | 12″F″ |
| B. Subtilis NRRL B—558 | 3.12 | 1.56 | 0.78 | 0.78 | 0.39 | 0.39 | 0.39 | 0.39 | 0.39 | 3.12 | 0.78 |
| M. luteus PCI 1001 | <0.2 | <0.2 | <0.2 | <0.2 | <0.2 | <0.2 | <0.2 | <0.2 | <0.2 | <0.2 | <0.2 |
| Staph. aureus Smith | 1.56 | 0.39 | 1.56 | 1.56 | 0.78 | 0.39 | 0.39 | <0.2 | 0.78 | 1.56 | 0.78 |
| Kl. pneumoniae PCI—602 | 3.12 | 1.56 | 3.12 | 3.12 | 6.25 | 1.56 | 3.12 | 3.12 | 3.12 | 3.12 | 1.56 |
| Sal. enteritidis 1891 | 3.12 | 1.56 | 3.12 | 3.12 | 3.12 | 3.12 | 3.12 | 3.12 | 3.12 | 3.12 | 3.12 |
| Pseud. aeruginosa A3 | 25 | 12.5 | 12.5 | 12.5 | 6.25 | 25 | 12.5 | 6.25 | — | 6.25 | 3.12 |

The compounds of this invention can be produced in the following manner:

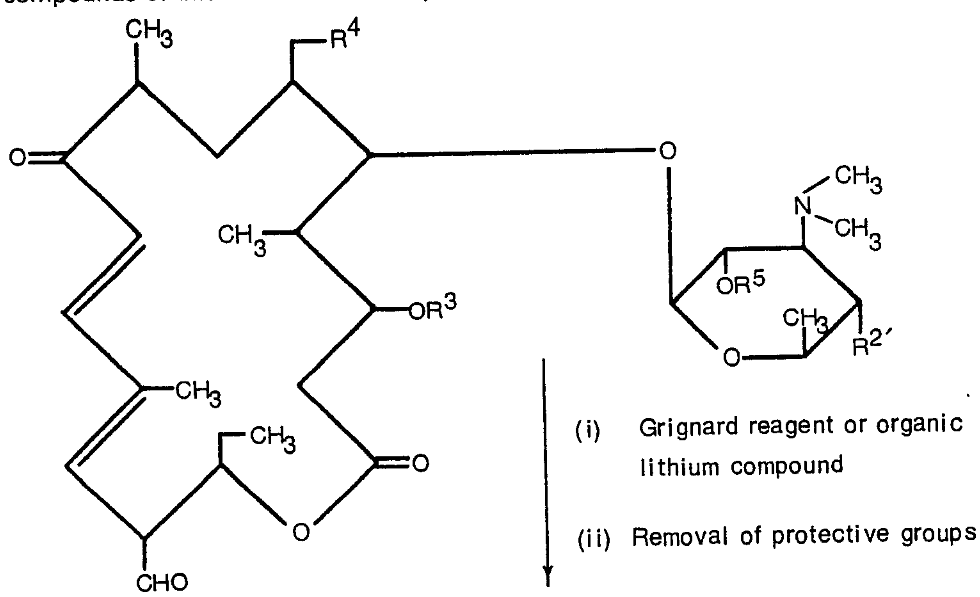


(II)

compound of formula (I).

In the above formula $R^{2'}$ represents a hydrogen atom or a protected hydroxyl group, $R^3$ represents a protective group for a hydroxyl group, $R^4$ represents a protected aldehyde group, $R^5$ represents a protective group for a hydroxyl group.

The compounds (I) can thus be produced by reacting 23-deoxy-23-oxo-mycaminosyl tylonolide derivatives of formula (II) with Grignard reagent of the formula: $R^1MgX$ (wherein $R^1$ is as defined above, and X represents a halogen atom) or with organic lithium compound of the formula: $R^1$—Li (wherein $R^1$ is as defined above) (step 1), and then releasing the hydroxyl- and aldehyde-protective groups (step 2).

Practical examples of protective groups are as follows:

Protective groups for a hydroxy group in the case of $R^{2'}$ and $R^5$: a lower acyl group such as an acetyl group, a propionyl group or a butyryl group.

Protective group for a hydroxyl group in the case of $R^3$: a tert-butyldimethylsilyl group, a 2-pyranyl group or a 2-furanyl group.

Protected aldehyde in the case of $R^4$: acetal or thioacetal; e.g. dimethylacetal, diethylacetal, diethylthioacetal, ethyleneacetal, ethylenethioacetal or propyleneacetal.

Examples of Grignard reagent of the formula: $R^1MgX$ are lower alkyl magnesium halide (such as methyl magnesium bromide, ethyl magnesium bromide or butyl magnesium bromide), aryl magnesium halide (such as phenyl magnesium bromide or naphthyl magnesium bromide), lower alkenyl magnesium halide (such as vinyl magnesium bromide or 2-propenyl magnesium bromide and lower alkinyl magnesium halide (such as ethinyl magnesium bromide or 2-propinyl magnesium bromide. Examples of the organic lithium compound of the formula $R^1$—Li are methyl lithium ($CH_3Li$), butyl lithium ($CH_3$—$CH_2$—$CH_2$—$CH_2Li$), ethylene lithium ($CH_2$=CHLi), 2-propene lithium, 2-propine and lithium, 2-phenyl lithium.

The reaction of the starting material (II) with the Grignard reagent or the organic lithium compound [step 1] is usually performed in an organic solvent (such as dry ether or tetrahydrofuran) at low temperature (for example, −50 to −80°C). The reaction can be completed within 1 to 3 hours.

The compounds thus formed are usually racemic compounds. If optical active isomers are desired, the formed products can be subjected to conventional procedures such as silica gel column chromatography to isolate optical isomers.

The compounds (I) can be obtained after releasing the protective groups [step 2]. The removal of $R^{2'}$ and $R^5$ (protective groups for 2′- and 4′-hydroxyl groups of mycaminose) is easily performed in conventional manner — for example, by heating in a suitable solvent such as methanol, a water-containing alcohol, or aprotic solvent containing water. The removal of $R^3$ (protective group for hydroxyl group) and the aldehyde protective group can be performed in a conventional manner, for example, by treatment with mineral acid such as hydrochloric acid or sulfuric acid or organic acid such as trifluoroacetic acid or trichloroacetic acid.

The invention is illustrated by the following Examples. The production methods for some of the starting materials for this invention are shown by the following Reference Examples. In these Examples NMR means a nuclear magnetic resonance spectrum and Me means a methyl group; the quoted chromatography eluant ratios are by volume.

The word "Kieselgel" used in some Examples is a Registered Trade Mark.

Reference Example 1

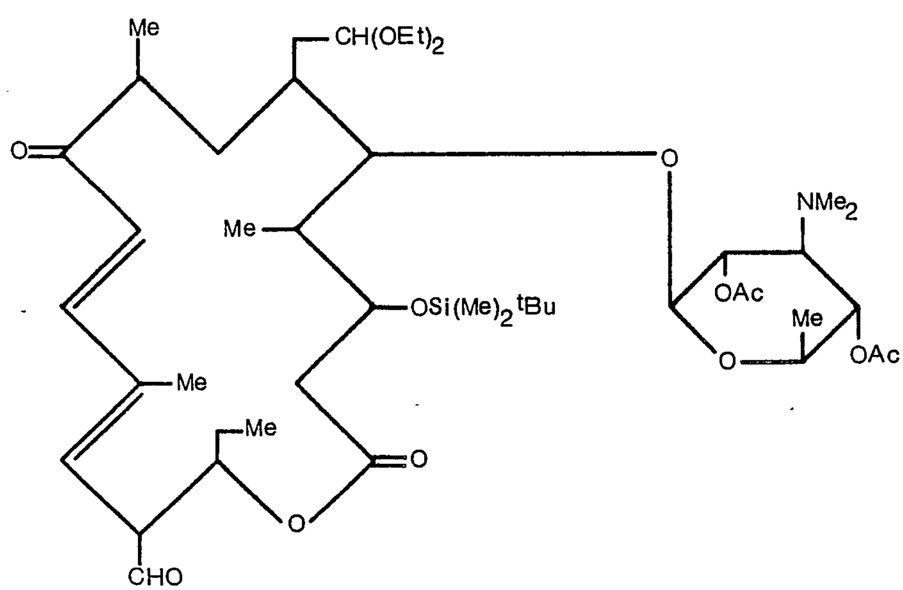

673 mg of 2',4'-di-O-acetyl-3-O-tert-butyldimethylsilyl mycaminosyl tylonolide diethylacetal was dissolved in 6.6 ml of dry benzene and 6.6 ml of dry dimethyl sulfoxide. After adding thereto 150 mg of pyridinium trifluoroacetate and 624 mg of N,N'-dicyclohexyl carbodiimide, the mixture was stirred at room temperature for 5 hours. The reaction mixture was added to a solution of 255 mg of oxalic acid dihydrate in 6.6 ml of dioxane, and the mixture was stirred for 1 hour at room temperature. The precipitates thus formed were removed by filtration and the reaction solution was concentrated under reduced pressure to give a syrupy material. The syrupy material was dissolved in 100 ml of benzene. The solution was washed with saturated aqueous sodium hydrogencarbonate and then with saturated aqueous sodium sulfate, and dried over anhydrous sodium sulfate. The solution was concentrated under reduced pressure to provide 700 mg of a solid material of 2',4'-di-O-acetyl-3-O-tert-butyldimethylsilyl-23-deoxy-23-oxo-mycaminosyl tylonolide diethylacetal. This product was used for the next reaction step, without purification.

Reference Example 2

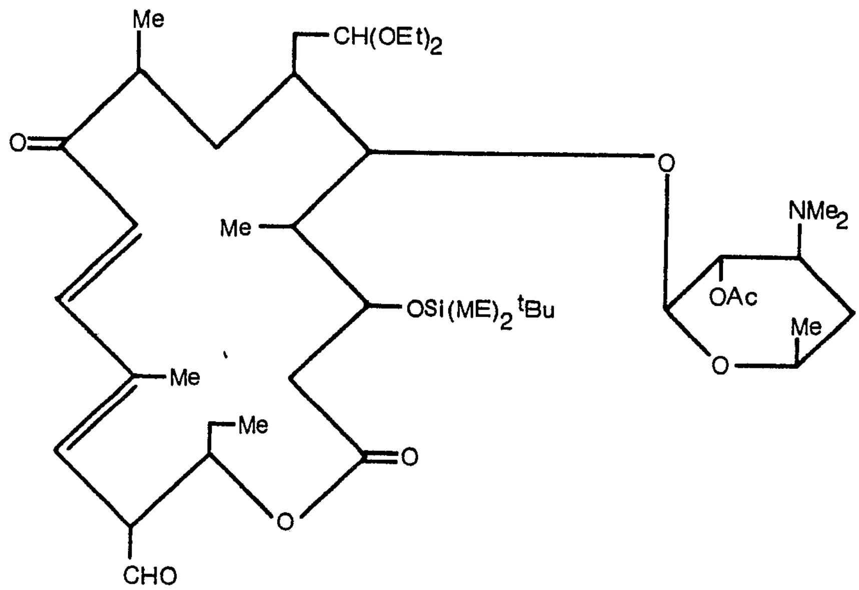

900 g of 2'-O-acetyl-3-O-tert-butyldimethylsilyl-4'-deoxymycaminosyl tylonolide diethyl acetal was dissolved in 9 ml of dry benzene and 9 ml of dry dimethyl sulfoxide, and after adding thereto 215 mg of pyridinium trifluoroacetate and 914 mg of N,N'-dicyclohexyl carbodiimide, the mixture was stirred at room temperature for 4 hours. The reaction mixture was poured into 9 ml of a solution of 372 mg of oxalic acid dihydrate in 9 ml of dioxane, and the mixture was stirred for 1 hour. Insoluble materials were removed by filtration, and the filtrate was concentrated under reduced pressure. A syrupy material obtained was dissolved in 100 ml of benzene, and the solution was washed with saturated aqueous sodium hydrogencarbonate and then with saturated aqueous sodium sulfate, dried over anhydrous sodium sulfate, and concentrated under reduced pressure to give 1 g of a solid material of 2'-O-acetyl-3-O-tert-butyldimethylsilyl-23,4'-dideoxy-23-oxo-mycaminosyl tylonolide diethylacetal. This product was used for the next reaction step, without purification.

Example 1 (1)

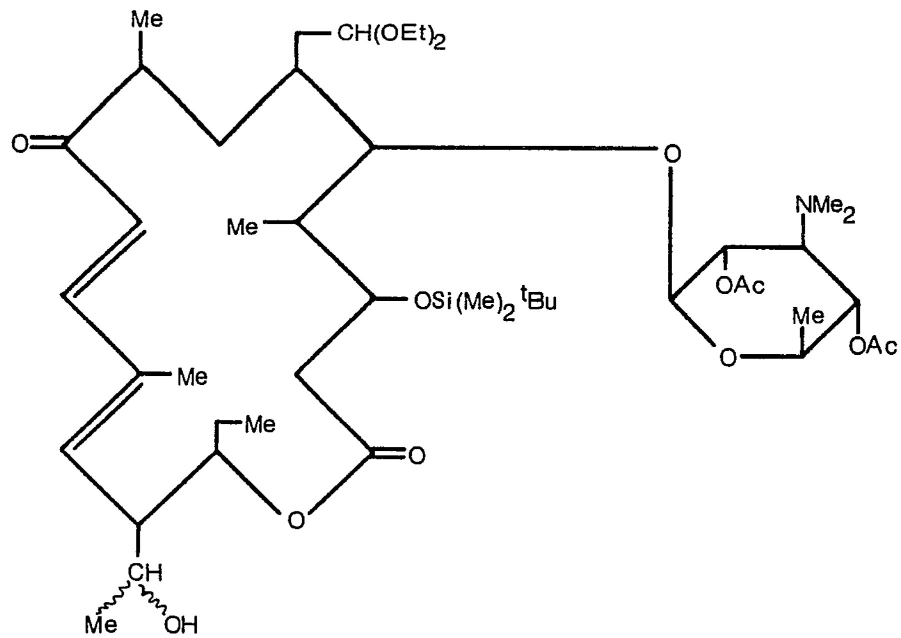

To a cold solution (−78°C) of 0.67 g of 2′,4′-O-acetyl-3-O-tert-butyldimethylsilyl-23-deoxy-23-oxo-mycaminosyl tylonolide diethylacetal was in 7 ml of dry tetrahydrofuran was added 1 ml of 1 M tetrahydrofuran solution of methyl magnesium bromide. It was kept for 3 hours at that temperature.

The reaction mixture was poured into saturated aqueous ammonium chloride, and extracted with 35 ml of chloroform. The extract was washed with saturated aqueous sodium sulfate, dried over anhydrous sodium sulfate, and concentrated under reduced pressure.

The residue was chromatographed (Kiesel gel-60: 70 g; toluene:ethyl acetate: 5:2—1:1) to give 230 mg of one optical isomer (A) and 180 mg of another optical isomer (B) of 2′,4′-di-O-acetyl-3-O-tert-butyldimethylsilyl-23-C-methylmycaminosyl tylonolide diethylacetal (these optical isomers are those caused by the 23-position asymmetric carbon atom).

NMR ($CDCl_3$)

| | | | |
|---|---|---|---|
| (A) | δ(ppm) | | |
| | 0.83 | 9H | 3—O—Si $(Me)_2$—$^t$Bu |
| | 1.12 | 3H | 23—C—Me |
| | 2.07 | 6H | 2′,4′—$OCOCH_3$ |
| | 23.5 | 6H | N—$Me_2$ |
| | 4.10 | 1H | 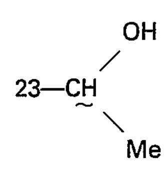 |

(B) δ(ppm)

| | | |
|---|---|---|
| 0.88 | 9H | 3—O—Si $(Me)_2$—[t]Bu |
| 1.21 | 3H | 23—C—Me |
| 2.08 | 6H | 2′,4′-$OCOCH_3$ |
| 2.36 | 6H | N—$me_2$ |
| 3.99 | 1H | 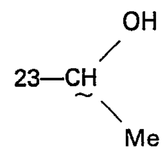 |

(2)

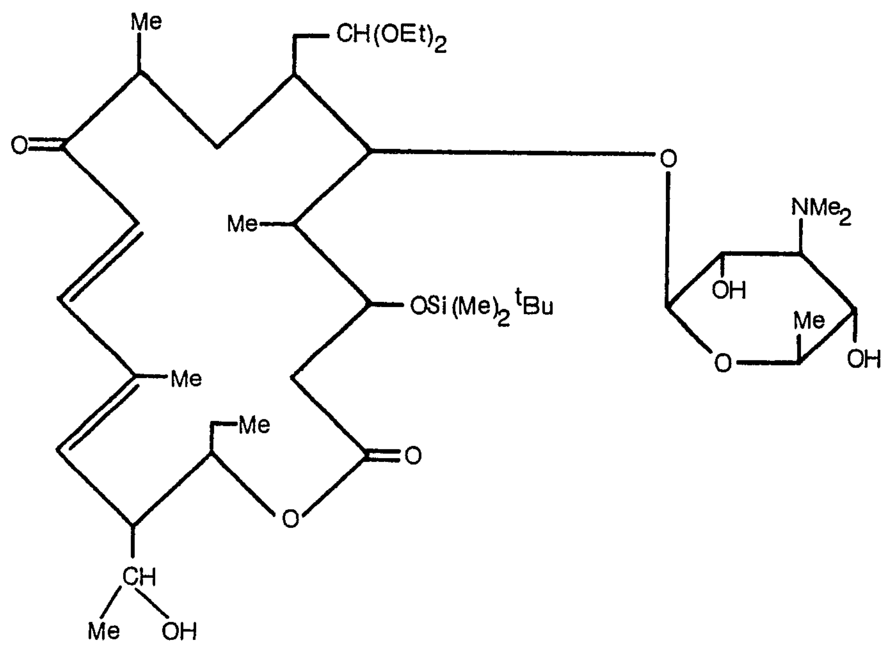

A solution of 230 mg of the optical isomer (A) of 2′,4′-di-O-acetyl-3-O-tert-butyldimethylsilyl-23-C-methylmycaminosyl tylonolide diethylacetal in 5 ml of methanol was kept at 50°C overnight. The reaction mixture was concentrated under reduced pressure to give 200 mg of 3-O-tert-butyldimethylsilyl-23-C-methylmycaminosyl tylonolide diethylacetal.

NMR ($CDCl_3$)

δ(ppm)

| | | |
|---|---|---|
| 0.88 | 9H | 3—O—Si $(Me)_2$—[t]Bu |
| 2.55 | 6H | N—$Me_2$ |
| 4.70 | 1H | H—23 |
| 6.07 | 1H | H—13 |

(3)

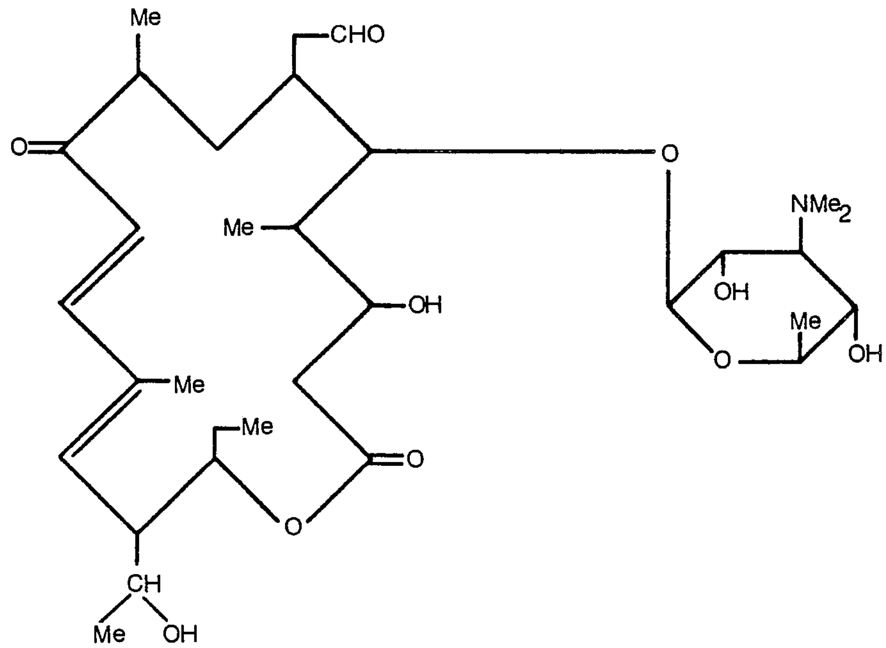

A solution of 200 mg of 3-O-tert-butyldimethylsilyl-23-C-methylmycaminosyl tylonolide diethylacetal (obtained at (2) above) in 1.2 ml of acetonitrile and 1.2 ml of 1N hydrochloric acid was kept at room temperature overnight. To the reaction mixture was added saturated aqueous sodium hydrogencarbonate (pH 9), and the mixture was extracted with 3 ml of chloroform and the solution washed with 3 ml of a saturated aqueous sodium sulfate solution, dried over anhydrous sodium sulfate, and concentrated under reduced pressure. The residue was chromatographed (Kiesel gel-60: 18g; chloroform:methanol:28% aqueous ammonia:10:1:0.1) to give 121 mg of 23-C-methylmycaminosyl tylonolide.

NMR ($CDCl_3$)

| δ(ppm) | | |
|---|---|---|
| 1.15 | 3H | 23—C—Me |
| 1.81 | 3H | 22—Me |
| 2.51 | 6H | N—$Me_2$ |
| 4.08 | 1H | H—23 |
| 4.26 | 1H | H—1′ |
| 5.20 | 1H | H—15 |
| 6.07 | 1H | H—13 |
| 9.69 | 1H | H—20 |

Example 2

(1)

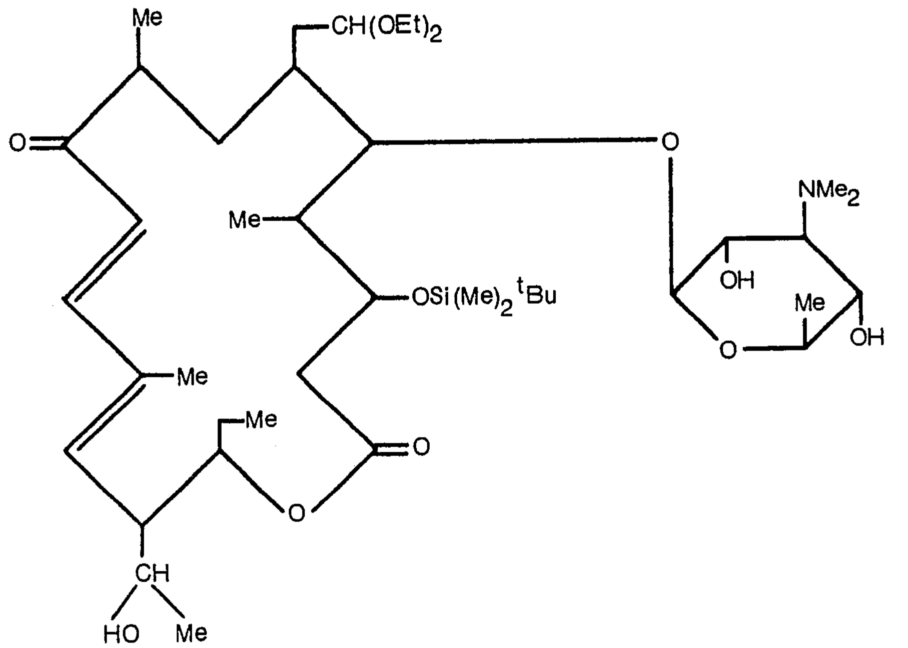

A solution of 114 mg of the optical isomer (B) obtained by Example 1 (1) of 2',4'-di-O-acetyl-3-O-tert-butyldimethylsilyl-23-C-methylmycaminosyl tylonolide diethylacetal in 2.2 ml of methanol was kept at 50°C overnight.

It was concentrated under reduced pressure, and the residue was chromatographed (Kiesel gel-60: 22 g; chloroform:methanol: 12:1—5:1) to give 70 mg of 3-O-tert-butyldimethylsilyl-23-C-methylmycaminosyl tylonolide diethylacetal.

NMR ($CDCl_3$)

| δ(ppm) | | |
|---|---|---|
| 0.88 | 9H | 3—O—Si $(Me)_2$—$^t$Bu |
| 2.55 | 6H | N—$Me_2$ |
| 4.01 | 1H | H—23 |
| 5.80 | 1H | H—13 |

(2)

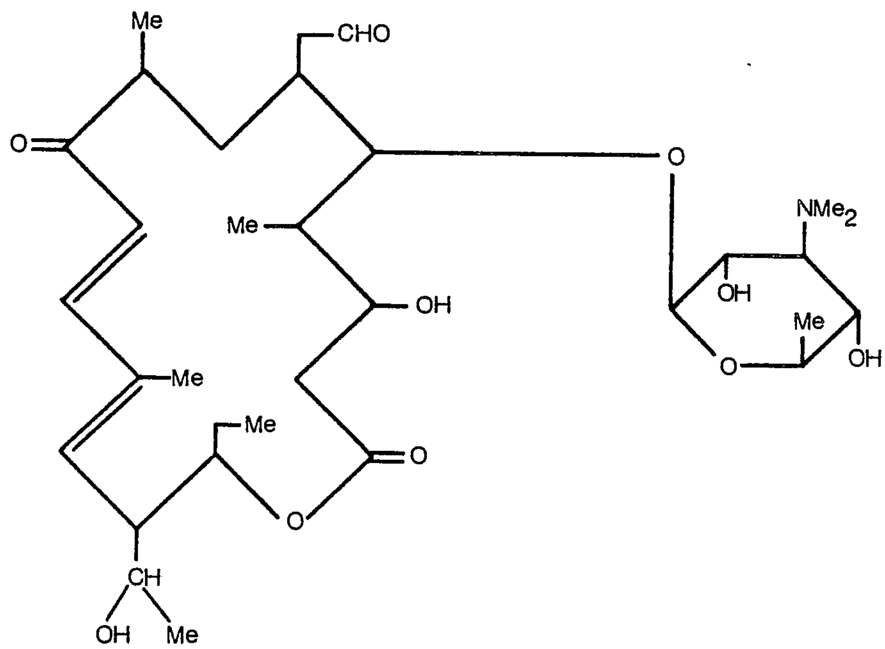

A solution of 53 mg of 3-O-tert-butyldimethylsilyl-23-C-methylmycaminosyl tylonolide diethylacetal (obtained at (1) above) in 0.27 ml of acetonitrile and 0.27 ml of 1N hydrochloric acid was kept at room temperature overnight. Usual work-up (cf. Ex.1(32)) gave a solid, which was chromatographed (Kiesel gel-60; chloroform:methanol:28% aqueous ammonia solution: 10:1:0.1) to give 22 mg of 23-C-methylmycaminosyl tylonolide diethylacetal).

NMR ($CDCl_3$)

| δ(ppm) | | |
|---|---|---|
| 1.23 | 3H | 23—C—Me |
| 1.83 | 3H | 22—Me |
| 2.50 | 6H | N—$Me_2$ |
| 3.98 | 1H | H—23 |
| 4.28 | 1H | H—1′ |
| 4.91 | 1H | H—15 |
| 5.76 | 1H | H—13 |
| 9.70 | 1H | H—20 |

Example 3

(1)

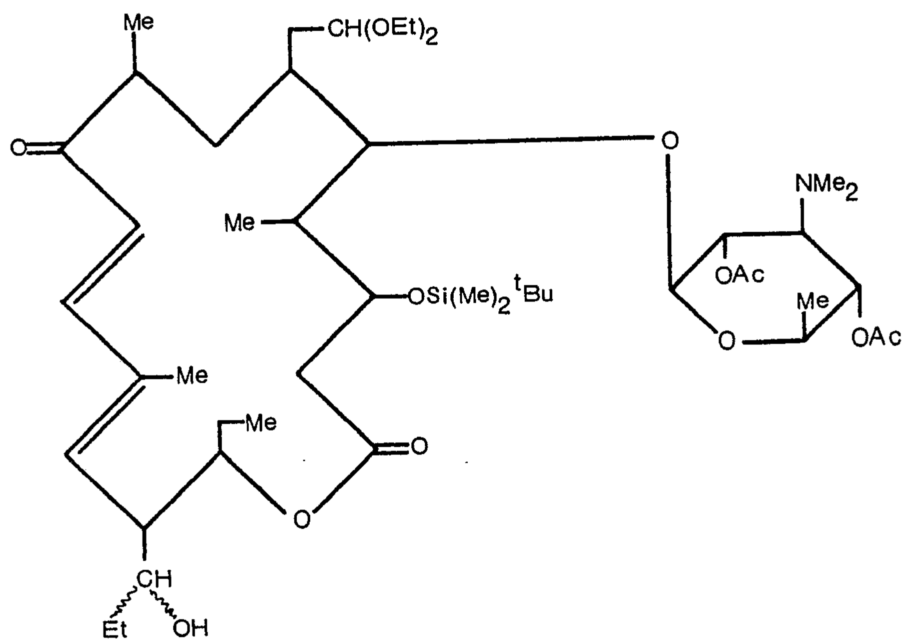

To a solution of 1.01 g 2',4'-di-O-acetyl-3-O-tert-butyldimethylsilyl-23-deoxy-23-oxo-mycaminosyl tylonolide diethylacetal in 10.5 ml of dry tetrahydrofuran, was added 0.89 ml of diethyl ether solution of ethyl magnesium bromide at −78°C. After 1 hour, the reaction mixture was poured into a saturated aqueous ammonium chloride solution. Usual workup (cf. Ex1(1)) gave a solid, which was chromatographed (Kiesel gel-60: 50 g; toluene:ethyl acetate: 3:1) to give 397.7 mg of one optical isomer (A) and 312.5 mg of another optical isomer (B) of 2',4'-di-O-acetyl-3-O-tert-butyldimethylsilyl-23-C-ethylmycaminosyl tylonolide diethylacetal (these optical isomers are those caused by the 23-position asymmetric carbon atom).

(2)

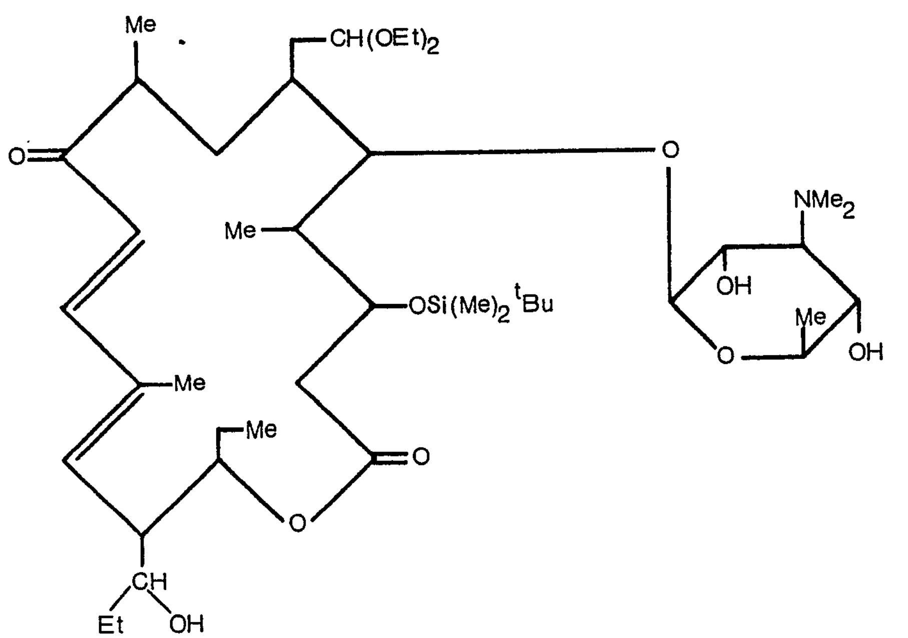

A solution of 397.7 mg of the optical isomer (A) obtained at (1) above of 2',4'-di-O-acetyl-3-O-tert-butyldimethylsilyl-23-C-ethylmycaminosyl tylonolide diethylacetal in 12 ml of methanol was kept at 50°C overnight. Usual work-up gave a solid, which was chromatographed (Kiesel gel-60: 50 g; chloroform:methanol:28% aqueous ammonia solution: 15:1:0.1) to give 271.3 mg of 3-O-tert-butyldimethylsilyl-23-C-ethylmycaminosyl tylonolide diethylacetal.

NMR ($CDCl_3$)
δ(ppm)

| | | |
|---|---|---|
| 0.87 | 9H | 3—O—Si $(Me)_2$—$^tBu$ |
| 2.56 | 6H | $NMe_2$ |
| 4.06 | 1H | H—23 |
| 6.00 | 1H | H—13 |

(3)

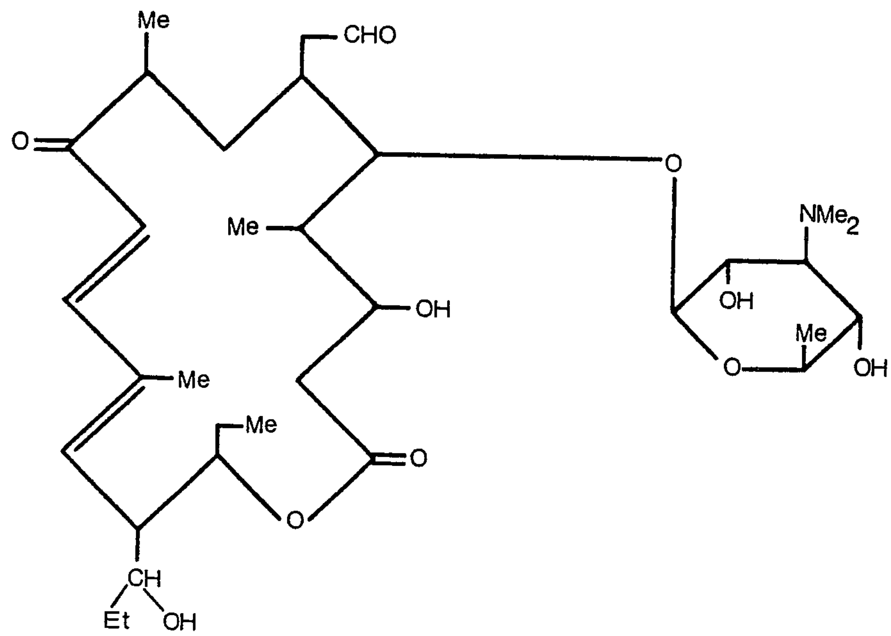

A solution of 60 mg of 3-O-tert-butyldimethylsilyl-23-C-ethylmycaminosyl tylonolide diethylacetal (obtained at (2) above) in 0.3 ml of acetonitrile and 0.3 ml of 1N hydrochloric acid was kept at 37°C overnight. Usual work-up (cf.Ex.1(3)) gave a solid which was chromatographed (Kiesel gel-60: 6 g, chloroform:methanol:28% aqueous ammonia solution: 20:1:0.1—15:1:0.1) to give 33.7 mg of 23-C-ethylmycaminosyl tylonolide.

NMR ($CDCl_3$)
δ(ppm)

| | | |
|---|---|---|
| 0.90 | 3H | 23—$CH_2CH_3$ |
| 1.42 | 2H | 23—$CH_2CH_3$ |
| 1.81 | 3H | 22—Me |
| 2.52 | 6H | $NMe_2$ |
| 4.27 | 1H | H—1' |
| 5.24 | 1H | H—15 |
| 6.06 | 1H | H—13 |
| 9.68 | 1H | H—20 |

Example 4

(1)

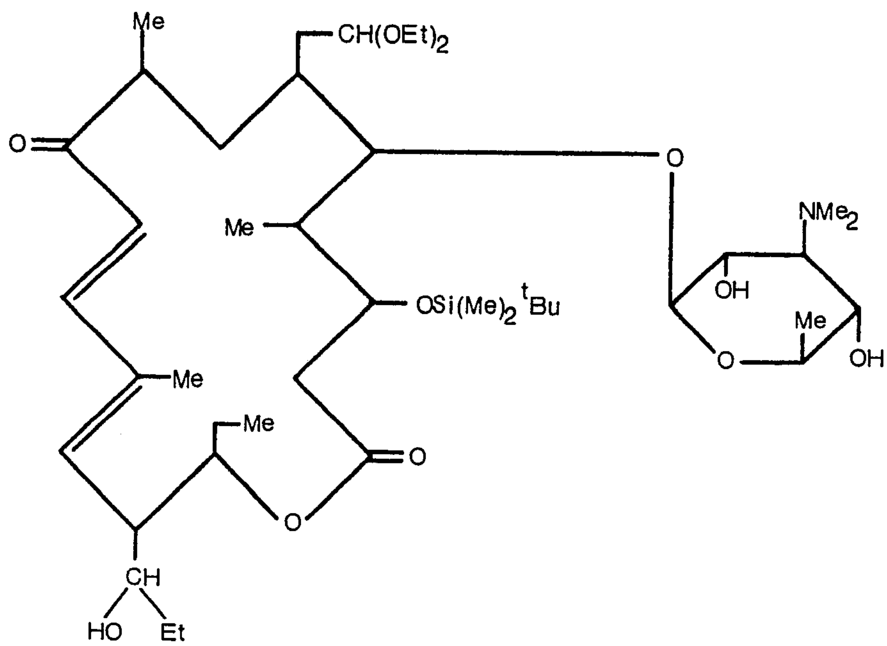

A solution of 312.5 mg of the optical isomer (B) obtained by example 3 (1) of 2′,4′-di-O-acetyl-3-O-tert-butyldimethylsilyl-23-C-ethylmycaminosyl tylonolide diethylacetal in 6.3 methanol was kept at 50°C overnight. Usual work-up gave a solid, which was chromatographed (Kiesel gel-60: 30 g; chloroform:methanol:28% aqueous ammonia solution: 18:1:0.1) to give 73.2 mg of 3-O-tert-butyldimethylsilyl-23-C-ethylmycaminosyl tylonolide diethylacetal.

| δ(ppm) | | |
|---|---|---|
| 0.87 | 9H | 3—O—Si $(Me)_2$—$^t$Bu |
| 2.56 | 6H | $NMe_2$ |
| 4.10 | 1H | H—23 |
| 5.75 | 1H | H—13 |

(2)

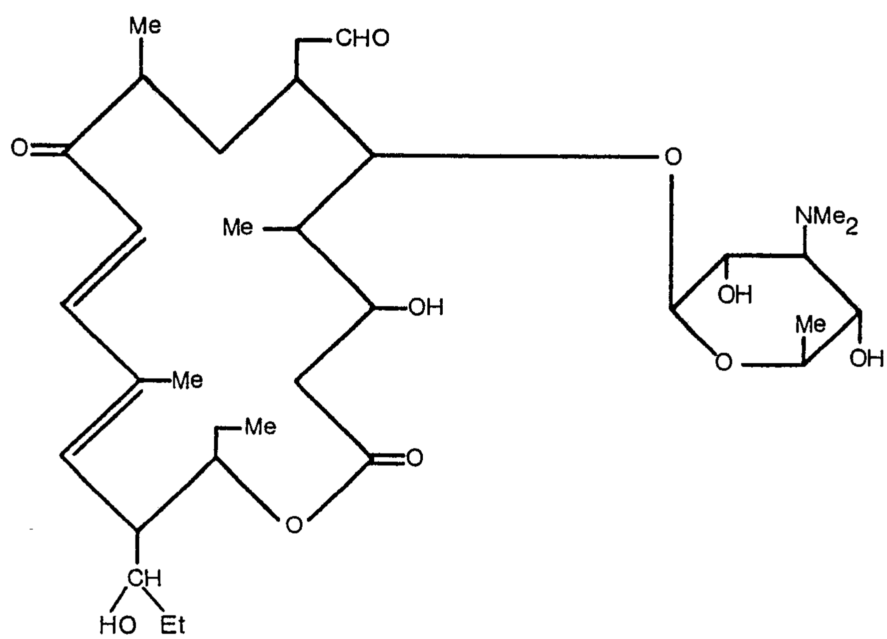

A solution of 46.4 mg of 3-O-tert-butyldimethylsilyl-23-C-ethylmycaminosyl tylonolide diethylacetal obtained at (1) above in 0.2 ml of acetonitrile and 0.2 ml of 1N hydrochloric acid was kept at room temperature for two days. Usual work-up (cf. Ex.2(2)) gave a solid, which was chromatographed (Kiesel gel-60: 6 g; chloroform:methanol:28% aqueous ammonia:20:1:0.1—15:1:0.1) to provide 8.8 mg of 23-C-ethylmycaminosyl tylonolide.

NMR ($CDCl_3$)

| | | |
|---|---|---|
| 0.99 | 3H | 23—$CH_2CH_3$ |
| 1.60 | 2H | 23—$CH_2CH_3$ |
| 1.81 | 3H | 22—Me |
| 2.51 | 6H | $NMe_2$ |
| 4.27 | 1H | H—1′ |
| 4.95 | 1H | H—15 |
| 5.76 | 1H | H—13 |
| 9.70 | 1H | H—20 |

Example 5

(1)

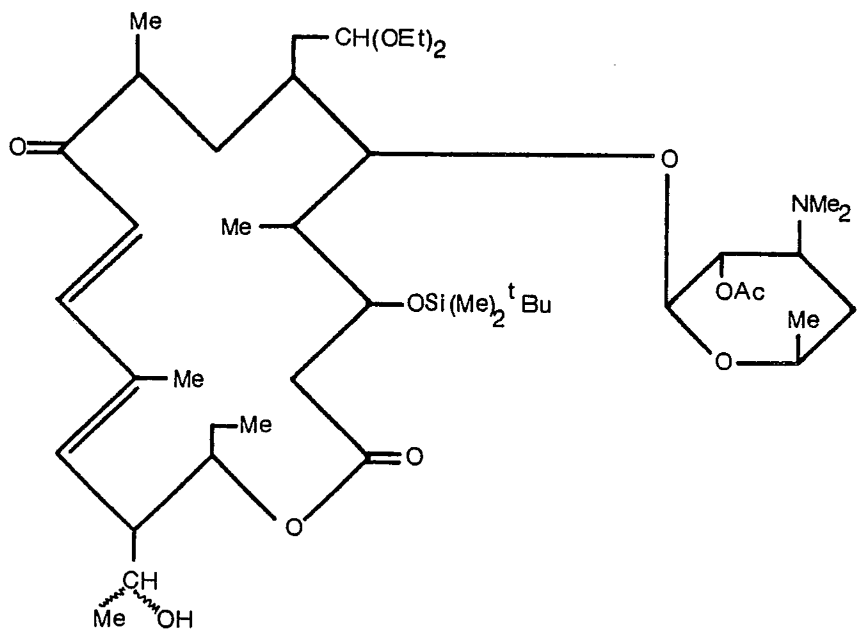

To a cold (−78°C) solution of 1 g of 2′-O-acetyl-3-O-tert-butyldimethylsilyl-23-,4′-dideoxy-23-oxo-mycaminosyltylonolide diethylacetal in dry tetrahydrofuran, was added 1.6 ml of 1m tetrahydrofuran solution of methyl magnesium bromide.

After 1 hour, the reaction mixture was poured into saturated aqueous ammonium chloride. Usual work-up (cf. Ex.1(1)) gave a solid, which was chromatographed (Kiesel gel-60: 90 g; toluene:acetone: 4:1—1:1) to give 380 mg of one optical isomer (A) and 160 mg of another optical isomer (B) of 2′-O-acetyl-3-O-tert-butyldimethylsilyl-4′-deoxy-23-C-methylmycaminosyl tylonolide diethylacetal (these isomers are those caused by the 23-position asymmetric carbon).

NME ($CDCl_3$)

(A)

| δ(ppm) | | |
|---|---|---|
| 0.86 | 9H | 3—O—Si $(Me)_2$—$^tBu$ |
| 1.12 | 3H | 23—C—Me |
| 2.10 | 3H | 2′—$OCOCH_3$ |
| 2.27 | 6H | $NMe_2$ |
| 4.10 | 1H | H—23 |
| 5.97 | 1H | H—13 |

(B)

| δ(ppm) | | |
|---|---|---|
| 0.85 | 9H | 3—O—Si $(Me)_2$—$^tBu$ |
| 1.19 | 3H | 23—C—Me |
| 2.09 | 3H | 2′—$OCOCH_3$ |
| 2.27 | 6H | $NMe_2$ |
| 3.98 | 1H | H—23 |
| 5.75 | 1H | H—13 |

(2)

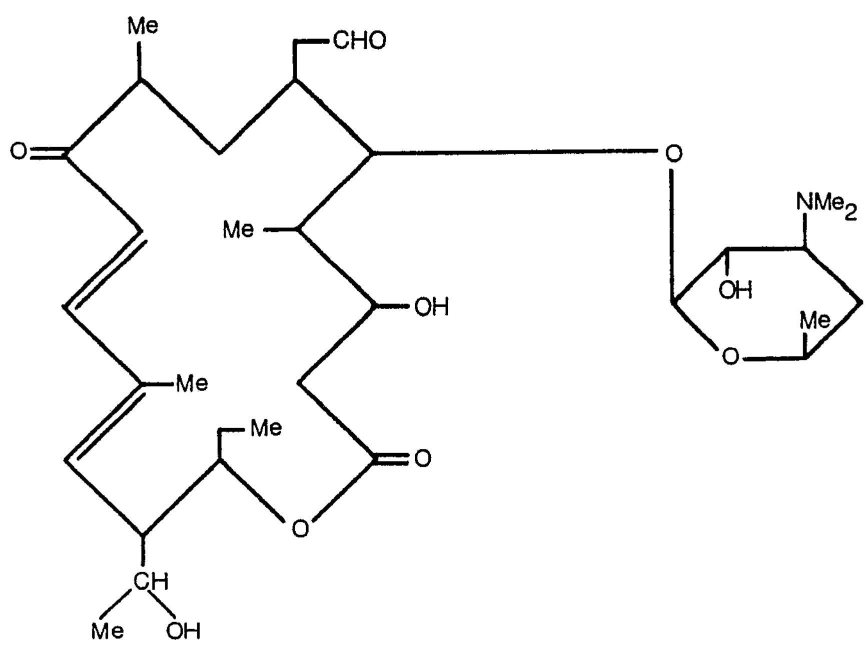

380 mg of the optical isomer (A) obtained at (1) above of 2′-O-acetyl-3-O-tert-butyldimethylsilyl-4′-deoxy-23-C-methylmycaminosyl tylonolide diethylacetal was subjected to methanolysis and hydrolysis as described before to give a solid, which was chromatographed (Kiesel gel-60: 10 g; chloroform:methanol:28% aqueous ammonia:15:1:0.1) to provide 130 mg of 4′-deoxy-23-C-methylmycaminosyl tylonolide.

NMR ($CDCl_3$)

| δ(ppm) | | |
|---|---|---|
| 1.83 | 3H | 22—Me |
| 2.27 | 6H | $NMe_2$ |
| 4.22 | 1H | H—1′ |
| 9.59 | 1H | CHO |

Example 6

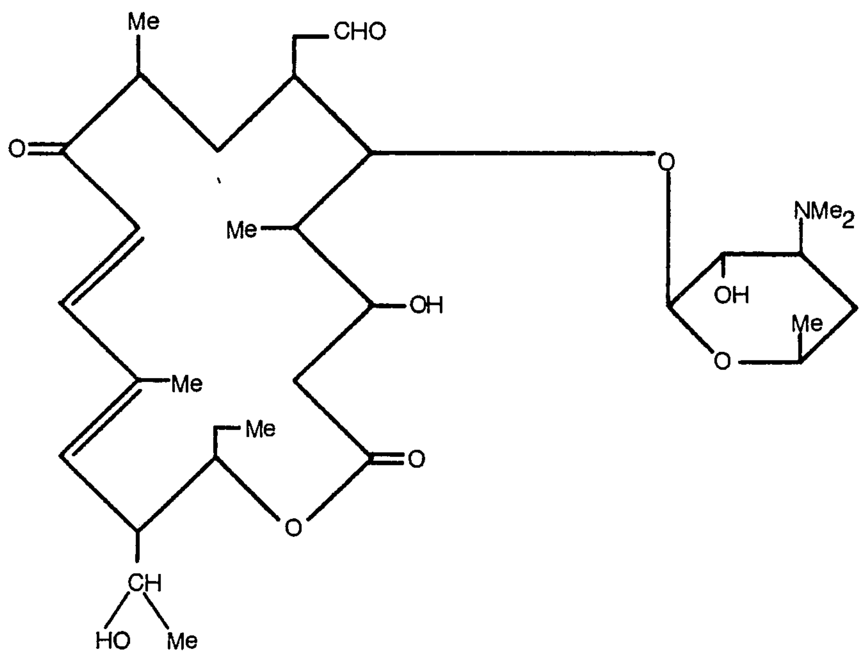

72 mg of the optical isomer (B) obtained by Example 5 (1) of 2′-O-acetyl-3-O-tert-butyldimethylsilyl-4′-deoxy-23-C-methylmycaminosyl tylonolide diethylacetal was treated as described for Example 5(2) to give a solid, which was chromatographed (Kiesel gel-60: 14 g; chloroform:methanol:20% aqueous ammonia:15:1:0.1) to provide 26 mg of 4′-deoxy-23-C-methylmycaminosyl tylonolide.

NMR ($CDCL_3$)

| δ(ppm) | | |
|---|---|---|
| 1.24 | 3H | 23—C—Me |
| 1.83 | 3H | 22—Me |
| 2.28 | 6H | $NMe_2$ |
| 3.99 | 1H | H—23 |
| 4.22 | 1H | H—1′ |
| 4.89 | 1H | H—15 |
| 5.80 | 1H | H—13 |
| 9.71 | 1H | H—20 |

Example 7

(1)

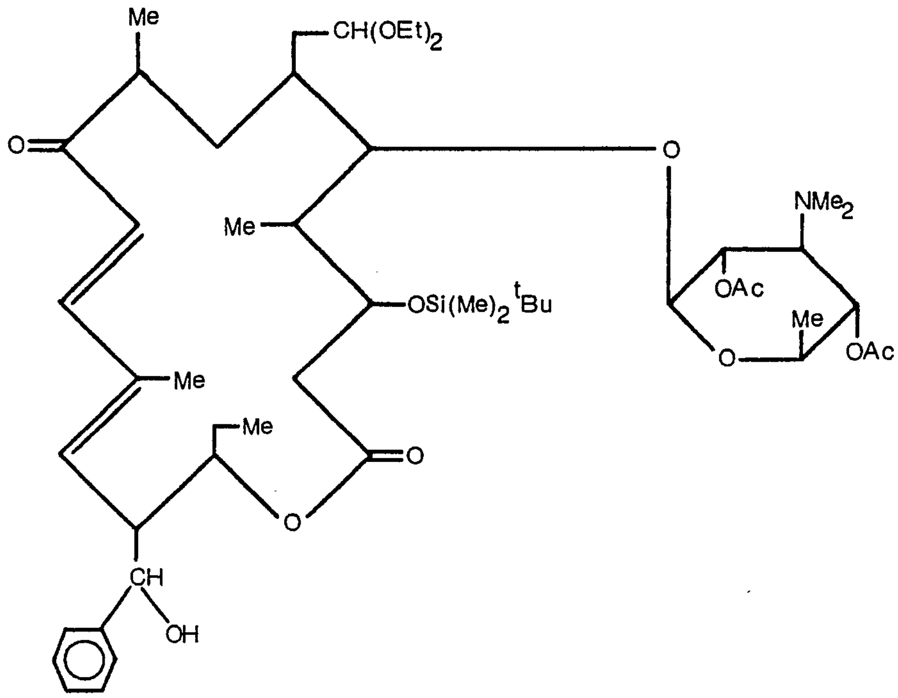

To a cold (−78°C) solution of 1 g of 2′,4′-di-O-acetal-3-O-tert-butyldimethylsilyl-23-deoxy-23-oxo-mycaminosyl tylonolide diethyl acetal in 10 ml of tetrahydrofuran, was added 1.2 ml of 2 M tetrahydrofuran solution of phenyl magnesium bromide, and it was kept at that temperature for 30 minutes. Usual work-up as described in Ex. (1) gave a solid, which was chromatographed (Kiesel gel-60: 50 g; toluene:ethyl acetate:4:1) to give 587 mg of 2′,4′-di-O-acetal-3-O-tert-butyldimethylsilyl-23-C-phenylmycaminosyl tylonolide diethylacetal.

NMR ($CDCl_3$)

| δ(ppm) | | |
|---|---|---|
| 0.9 | 9H | 3—O—Si $(Me)_2$—$^tBu$ |
| 2.1 | 6H | 2′,4′—$OCOCH_3$ |
| 2.35 | 6H | $NMe_2$ |
| 7.25 | ~5H | 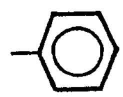 |

(2)

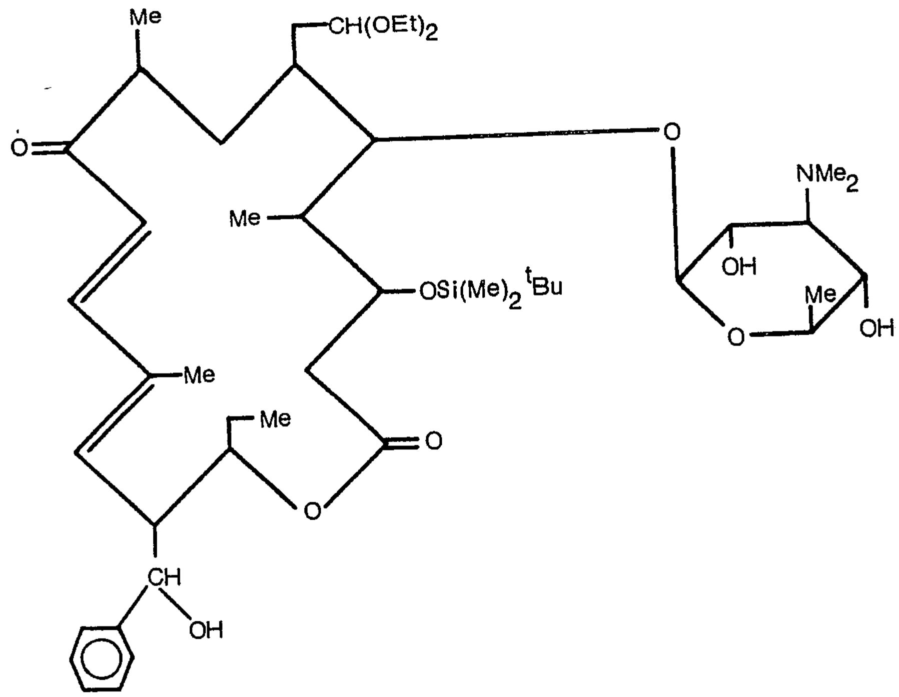

A solution of 432 mg of 2',4'-di-O-acetyl-3-O-butyldimethylsilyl-23-C-phenylmycaminosyl tylonodile diethylacetal in 8.65 ml of methanol was kept at 50°C overnight. Usual work-up gave 137 mg of 3-O-tert-butyldimethylsilyl-23-C-phenylmycaminosyl tylonolide diethylacetal.

NMR $(CDCl)_3$

| δ (ppm) | | |
|---|---|---|
| 0.93 | 9H | 3—O—$Si(Me)_2$ ${}^tBu$ |
| 2.50 | 6H | $NMe_2$ |
| 4.37 | 1H | H—1' |
| 7.25 | 5H | 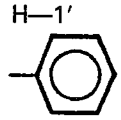 |

(3)

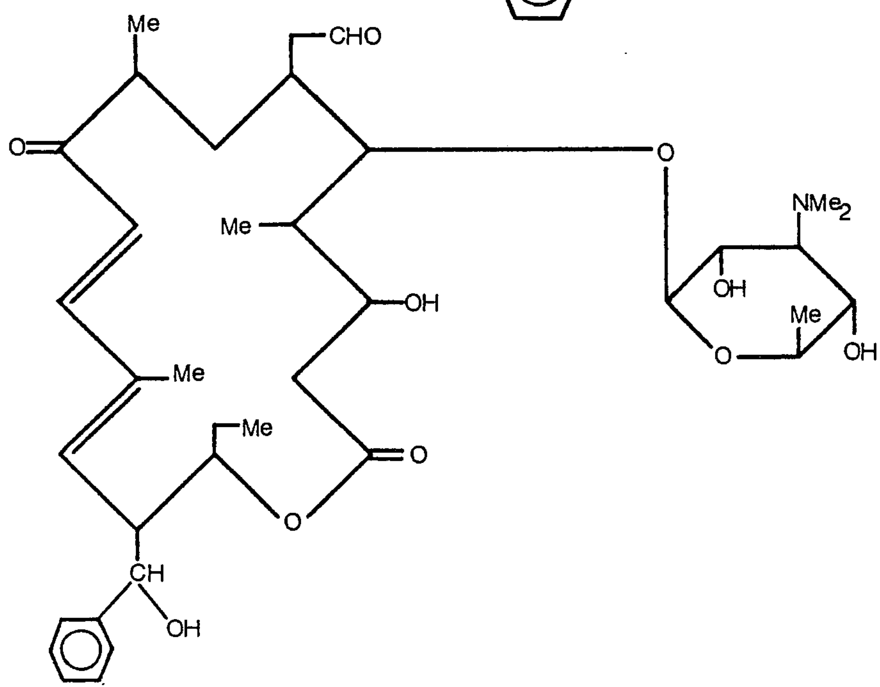

A solution of 137 mg of 3-O-tert-butyldimethylsilyl-23-C-phenylmycaminosyl tylonolide diethylacetal in 0.69 ml of acetonitrile and 0.69 ml of 1N hydrochloric acid was kept at room temperature overnight. Usual work-up as described in Ex. 1(3) gave, after chromatography (Kiesel gel-60: 13 g; chloroform:methanol: 28% aqueous ammonia: 15:1:0.1—12:1:0.1), 65.5 mg of 23-C-phenylmycaminosyl tylonolide.

NMR ($CDCl_3$)

| δ(ppm) | | |
|---|---|---|
| 2.50 | 6H | $NMe_2$ |
| 4.32 | 1H | H—1′ |
| 5.47 | 1H | H—13 |
| 7.25 | 5H | 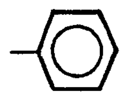 |
| 9.74 | 1H | CHO |

Example 8

(1)

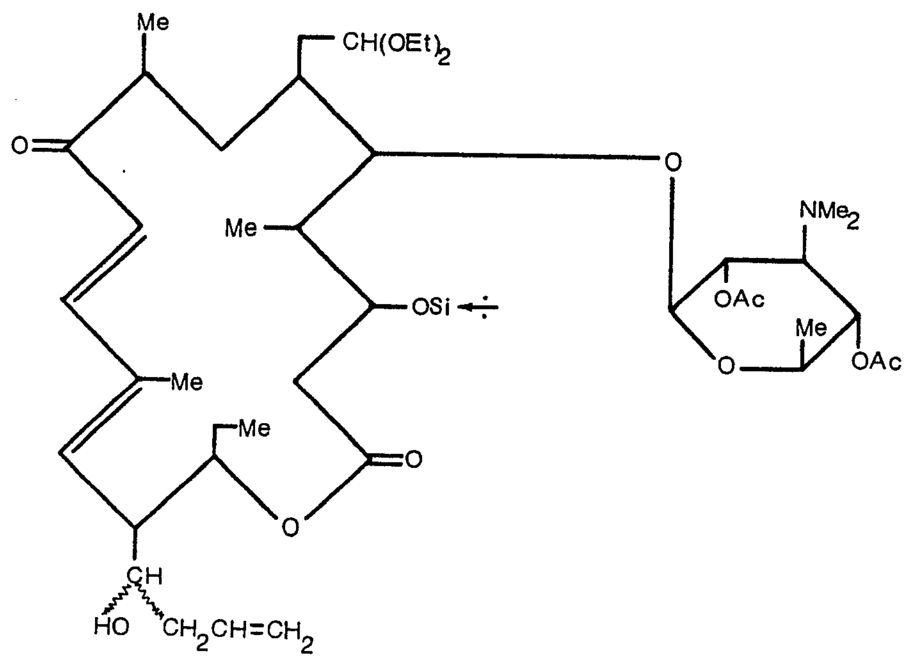

To a cold (−78°C solution of 782 mg of 2′,4′-di-O-acetyl-3-O-tert-butyl-dimethylsilyl-23-deoxy-23-oxo-mycaminosyl tylonolide diethylacetal in 10 ml of tetrahydrofuran, was added 2.5 ml. of 1M ether solution of allyl magnesium bromide. Usual work-up as described in Ex. 1(1) gave, after chromatography (toluene:ethyl acetate: 2:1 as eluant), 201 mg of one optical isomer (A) and 119 mg of another optical isomer (B) of 2′,4′-di-O-acetyl-3-O-tert-butyldimethylsilyl-23-C-(2-propenyl)mycaminosyl tylonolide diethylacetal.

NMR ($CDCl_3$)

(A)

| δ(ppm) | | |
|---|---|---|
| 0.90 | 9H | Si—tBu |
| 2.10 | 6H | 2′.4′—OCOMe |
| 2.40 | 6H | $NMe_2$ |
| 4.41 | 1H | H—1′ |

| | | | |
|---|---|---|---|
| (B) | δ(ppm) | | |
| | 0.90 | 9H | Si—tBu |
| | 2.10 | 6H | 2′.4′—OCOMe |
| | 2.40 | 6H | $nMe_2$ |
| | 6.32 | 1H | H—10 |

(2)

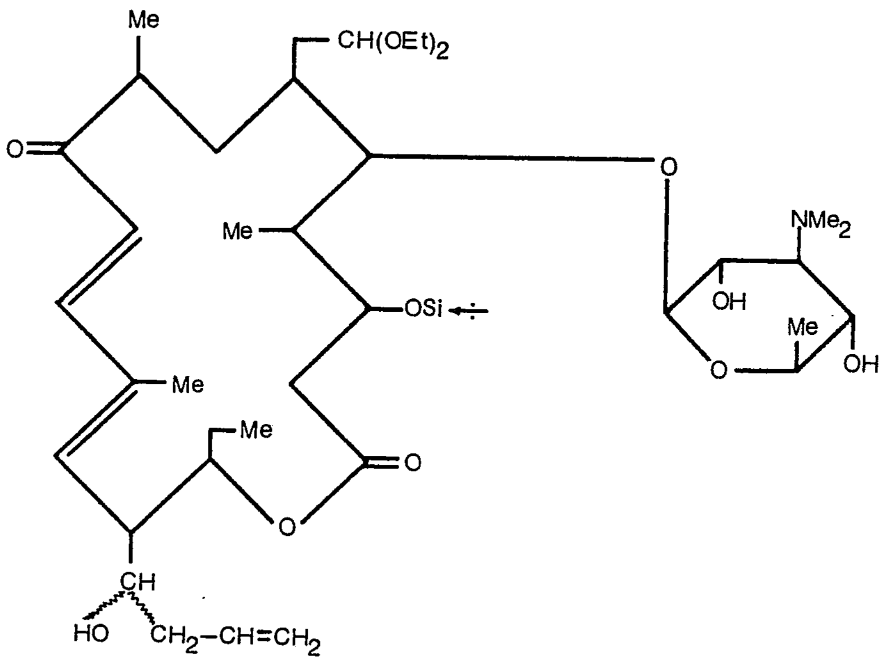

201 mg of the optical isomer (A) and 119 mg of the optical isomer (B) obtained at (1) above of 2′,4′-di-O-acetyl-3-O-tert-butyl-dimethylsilyl-23-C-(2-propenyl)mycaminosyl tylonolide diethylacetal were subjected to methanolysis to give, after chromatography (chloroform:methanol: 28% aqueous ammonia: 30:1:0.1), respectively 66 mg of one optical isomer (C) and 60 mg of another optical isomer (D) of 3-O-tert-butyldimethylsilyl-23-C-(2-propenyl)mycaminosyl tylonolide diethyl acetal as sold material.

NMR ($CDCl_3$)

| | | | |
|---|---|---|---|
| (C) | δ(ppm) | | |
| | 0.90 | 9H | Si—tBu |
| | 1.86 | 3H | 22—Me |
| | 2.55 | 6H | $NMe_2$ |
| | 4.33 | 1H | H—1′ |
| | 6.27 | 1H | H—10 |
| (D) | δ(ppm) | | |
| | 0.90 | 9H | Si—tBu |
| | 1.83 | 3H | 22—Me |
| | 2.57 | 6H | $NMe_2$ |
| | 4.33 | 1H | H—1′ |
| | 6.10 | 1H | H—13 |

(3)

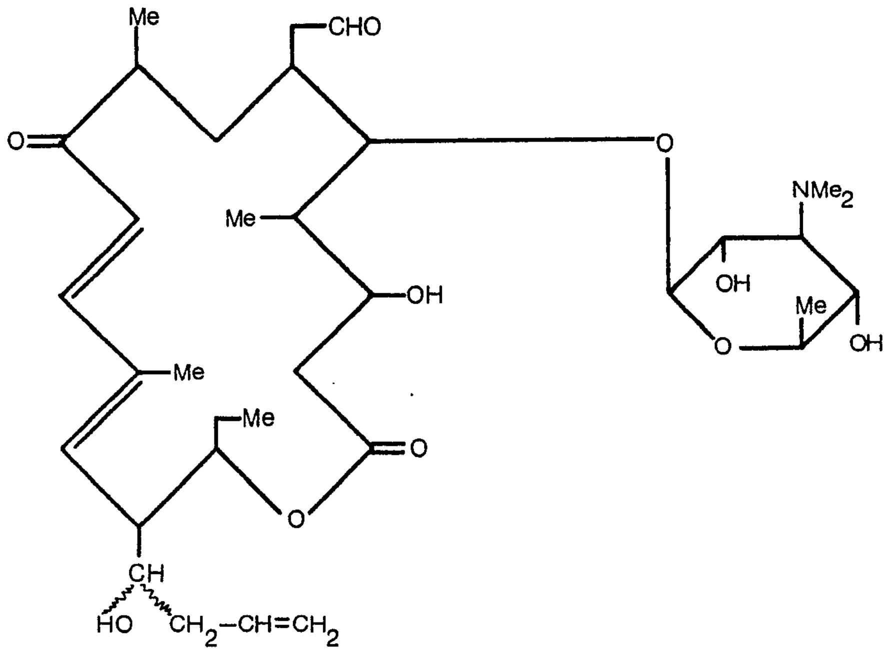

66 mg of the optical isomer (C) and 60 mg of the optical isomer (D) of 3-O-tert-butylmethylsilyl-23-C-(2-propenyl)mycaminosyl tylonolide diethylacetal were hydrolysed. Usual work-up gave, after chromatography (chloroform:methanol: 28% aqueous ammonia: 20:1:0.1), respectively 40 mg of one optical isomer (E) and 39 mg of another optical isomer (F) of 23-C-(2-propenyl)mycaminosyl tylonolide as solid material.

NMR ($CDCl_3$)

| | δ(ppm) | | |
|---|---|---|---|
| (E) | | | |
| | 1.79 | 3H | 22—Me |
| | 2.50 | 6H | $NMe_2$ |
| | 4.27 | 1H | H—1 |
| | 5.04~5.18 | 2H | H—25a.b. |
| | 5.65~5.82 | 1H | H—25 |
| | 6.11 | 1H | H—13 |
| | 6.30 | 1H | H—10 |
| | 7.40 | 1H | H—11 |
| | 9.69 | 1H | H—20 |
| (F) | δ(ppm) | | |
| | 1.83 | 3H | 22—Me |
| | 2.51 | 6H | $NMe_2$ |
| | 4.29 | 1H | H—1′ |
| | 4.98 | 1H | H—15 |
| | 5.13~5.23 | 2H | H—25a.b |
| | 56.5~5.87 | 2H | H—13 C H—25 |
| | 6.25 | 1H | H—10 |
| | 7.26 | 1H | H—11 |
| | 9.70 | 1H | H—20 |

Example 9

(1)

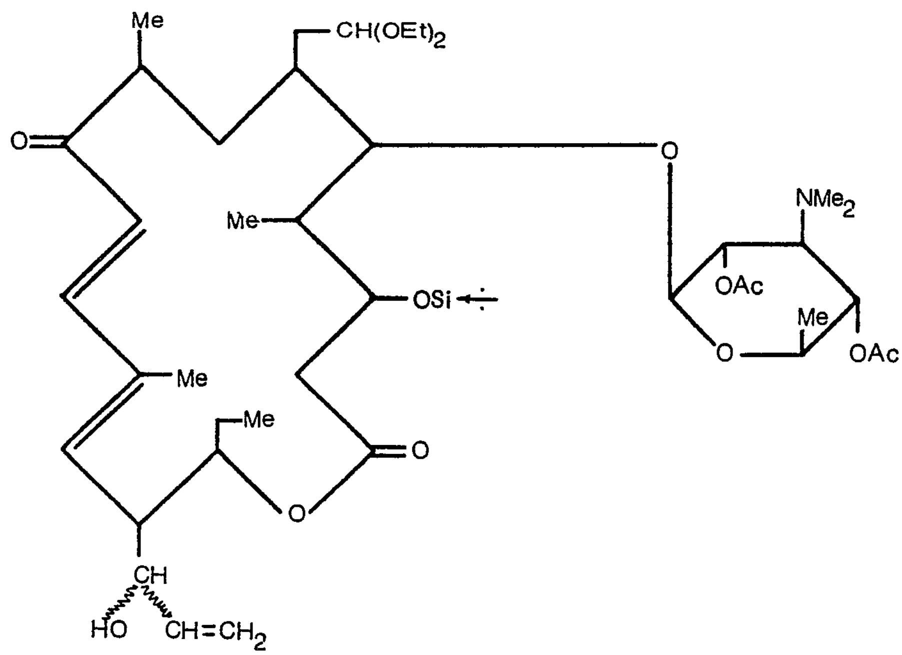

To a cold (−78°C) solution of 903 mg of 2′-4′-di-O-acetyl-3-O-tert-butyl-dimethylsilyl-23-deoxy-23-oxo-mycaminosyl tylonolide diethylacetal, was added vinyl magnesium bromide (2M tetrahydrofuran solution), and usual work-up gave, after chromatography, 422 mg of one optical isomer (A) and 166 mg of another optical isomer (B) of 2′,4′-di-O-acetyl-3-O-tert-butyldimethylsilyl-23-C-vinylmycaminosyl tylonolide diethylacetal as solid material.

NMR ($CDCl_3$)

| | | | |
|---|---|---|---|
| (A) | δ(ppm) | | |
| | 0.91 | 9H | Si—tBu |
| | 2.11 | 6H | 2′.4′—OCOMe |
| | 2.40 | 6H | $NMe_2$ |
| | 4.42 | 1H | H—1′ |
| | 6.30 | 1H | H—10 |
| (B) | δ(ppm) | | |
| | 0.90 | 9H | Si—tBu |
| | 2.10 | 6H | 2′.4′—OCOMe |
| | 2.40 | 6H | $NMe_2$ |
| | 6.31 | 1H | H—10 |

(2)

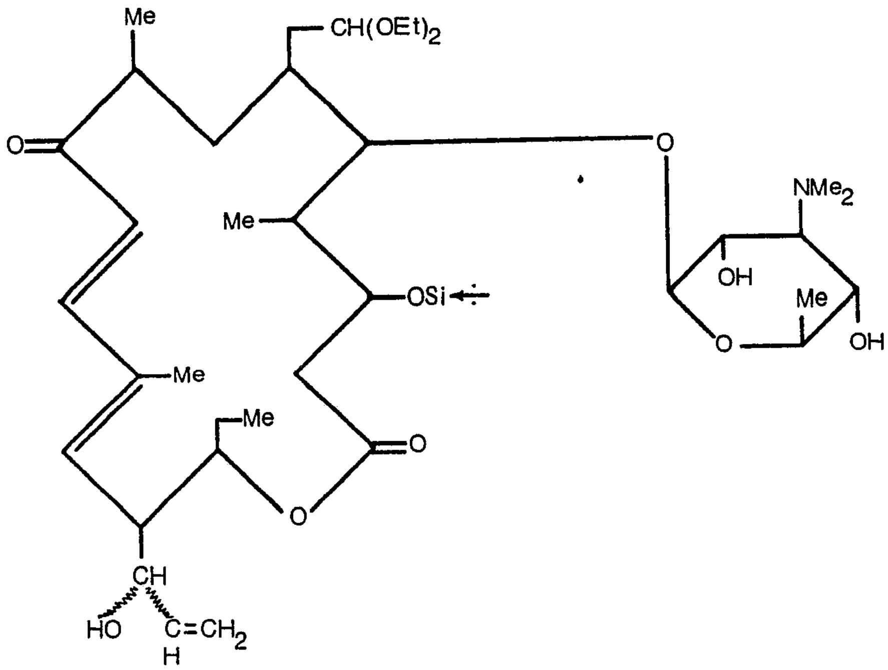

422 mg of the isomer (A) and 166 mg of the isomer (B) of 2,4-di-O-acetyl-3-O-tert-butyl dimethylsilyl-23-C-vinylmycaminosyl tylonolide diethylacetal were treated as an Example 8 (2) to give respectively 350 mg of one isomer (C) and 73 mg of another isomer (D) of 3-O-tert-butyldimethylsilyl-23-C-vinylmycaminosyl tylonolide diethylacetal as solid material.

NMR ($CDCl_3$)

| | | | |
|---|---|---|---|
| (C) | δ(ppm) | | |
| | 0.87 | 9H | Si—tBu |
| | 2.53 | 6H | $NMe_2$ |
| | 4.30 | 1H | H—1′ |
| | 6.28 | 1H | H—10 |
| (D) | δ(ppm) | | |
| | 0.90 | 9H | Si—tBu |
| | 2.55 | 6H | $NMe_2$ |
| | 6.30 | 1H | H—10 |
| | 7.25 | 1H | H—11 |

(3)

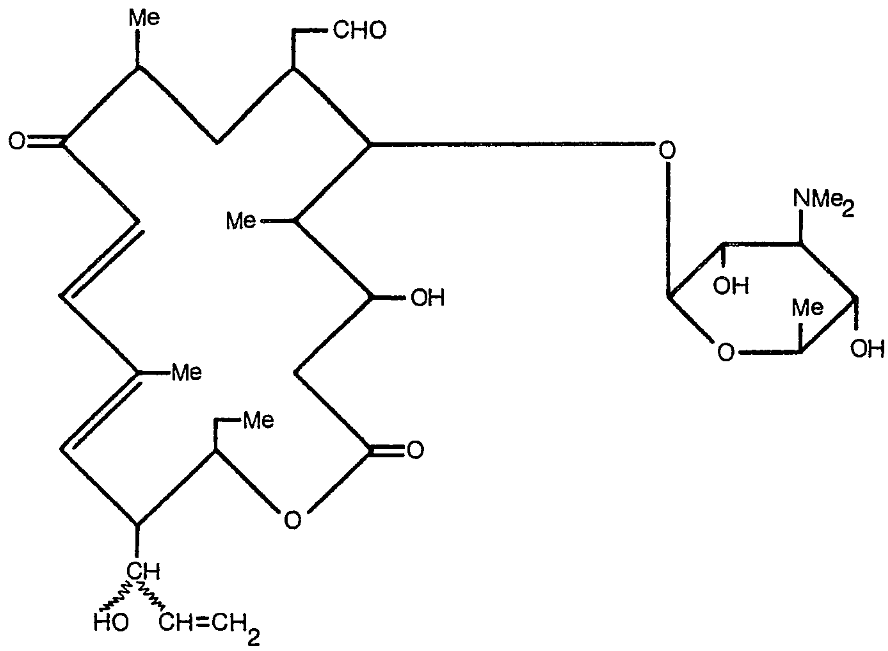

350 mg of the isomer (C) and 73 mg of the isomer (D) of 3-O-tert-butyldimethylsilyl-23-C-vinylmycaminosyl tylonolide diethylacetal were treated as in Example 8 (3) to give respectively 186 mg of one isomer (E) and 45 mg of another isomer (F) of 23-C-vinylmycaminosyl tylonolide as solid material.

NMR ($CDCl_3$)

| | | | |
|---|---|---|---|
| (E) | δ(ppm) | | |
| | 1.78 | 3H | 22—Me |
| | 2.52 | 6H | $NMe_2$ |
| | 4.28 | 1H | H—1′ |
| | 4.39 | 1H | H—23 |
| | 5.14 | 1H | H—25a |
| | 5.2~5.3 | 2H | H—15 C H—25b |
| | 5.77 | 1H | H—24 |
| | 6.05 | 1H | H—13 |
| | 6.27 | 1H | H—10 |
| | 7.35 | 1H | H—11 |
| | 9.69 | 1H | H—20 |

| (F) | δ(ppm) | | |
|---|---|---|---|
| | 1.84 | 3H | 22—Me |
| | 2.51 | 6H | $NMe_2$ |
| | 4.25~4.34 | 2H | H—1′ C H—23 |
| | 4.92 | 1H | H—15 |
| | 5.27~5.36 | 2H | H—25a,b |
| | 5.76 | 1H | H—13 |
| | 5.92 | 1H | H—24 |
| | 6.27 | 1H | H—10 |
| | 7.29 | 1H | H—11 |
| | 9.70 | 1H | H—20 |

## Example 10

(1)

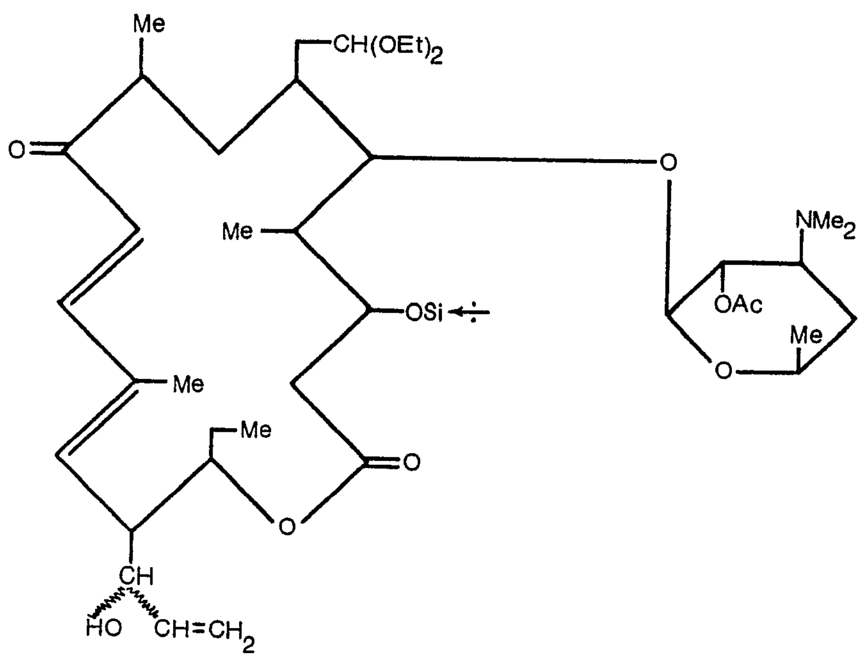

703 mg of 2′-O-acetyl-3-O-tert-butyldimethylsilyl-4′-deoxymycaminosyl tylonolide diethylacetal was treated as in Example 9 (1) to give 300 mg of one isomer (A) and 105 mg of another isomer (B) of 2′-O-acetyl-3-O-tert-butylmethylsilyl-4′-deoxy-23-C-vinylmycaminosyl tylonolide diethylacetal as solid material.

NMR ($CDCl_3$)

| (A) | δ(ppm) | | |
|---|---|---|---|
| | 0.88 | 9H | Si—tBu |
| | 2.11 | 3H | 2′—OCOMe |
| | 2.30 | 6H | $NMe_2$ |
| | 6.00 | 1H | H—13 |
| | 6.29 | 1H | H—10 |

| | | | |
|---|---|---|---|
| (B) | δ(ppm) | | |
| | 0.88 | 9H | Si—tBu |
| | 2.10 | 3H | 2′—OCOMe |
| | 2.29 | 6H | $NMe_2$ |
| | 5.71 | 1H | H—13 |
| | 6.27 | 1H | H—10 |

(2)

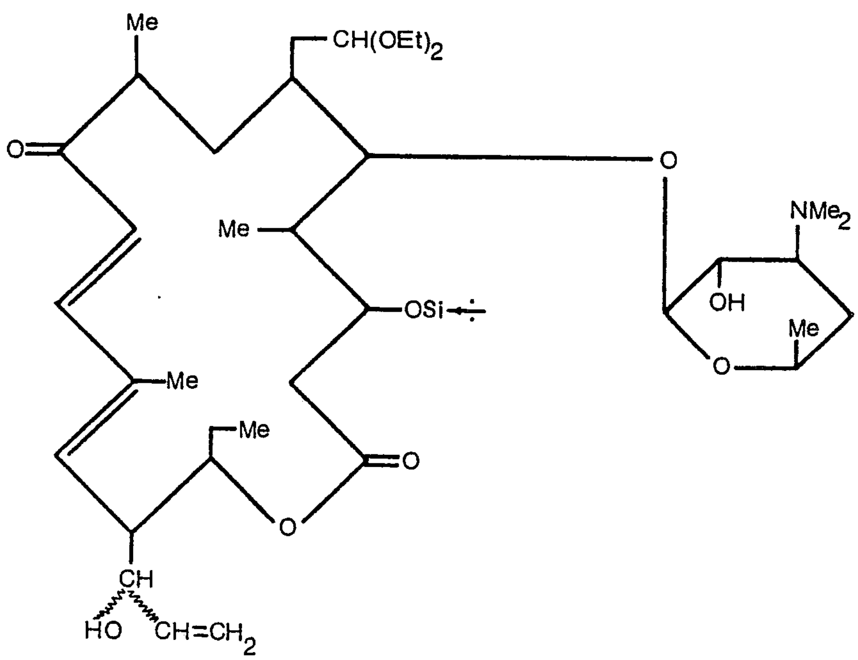

300 mg of the isomer (A) and 105 mg of the isomer (B) of 2′-O-acetyl-3-O-tert-butyldimethylsilyl-4′-deoxy-23-C-vinylmycaminosyl tylonolide diethylacetal were treated as in Example 8 (2) to give respectively 175 mg of one optical isomer (C) and 65 mg of another optical isomer (D) of 3-O-tert-butyldimethylsilyl-4′-deoxy-23-C-vinylmycaminosyl tylonolide diethylacetal as solid material.

NMR ($CDCl_3$)

| | | | |
|---|---|---|---|
| (C) | δ(ppm) | | |
| | 0.90 | 9H | Si—tBu |
| | 2.33 | 6H | $NMe_2$ |
| | 4.77 | 1H | H—20 |
| | 6.30 | 1H | H—10 |
| (D) | δ(ppm) | | |
| | 0.91 | 9H | Si—tBu |
| | 2.32 | 6H | $NMe_2$ |
| | 6.31 | 1H | H—10 |
| | 7.27 | 1H | H—11 |

(3)

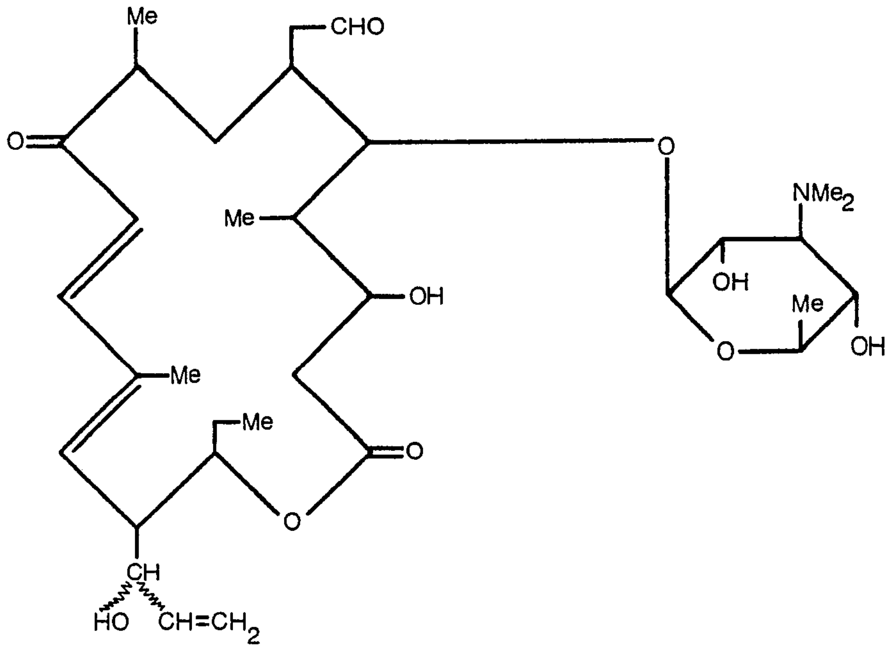

175 mg of the isomer (C) and 65 mg of the isomer (D) of 3-O-tert-butyldimethylsilyl-4'-deoxy-23-C-vinylmycaminosyl tylonolide diethylacetal were treated as in Example 8 (3) to give respectively 86 mg of one isomer (E) and 41 mg of another isomer (F) of 4'-deoxy-23-C-vinylmycaminosyl tylonolide as solid material.

NMR ($CDCl_3$)

| | | | |
|---|---|---|---|
| (E) | δ(ppm) | | |
| | 1.76 | 3H | 22—Me |
| | 2.27 | 6H | $NMe_2$ |
| | 4.22 | 1H | H—1' |
| | 4.41 | 1H | H—23 |
| | 5.1~5.3 | 3H | H—25a,b, H—1b |
| | 5.77 | 1H | H—24 |
| | 6.09 | 1H | H—13 |
| | 6.30 | 1H | H—10 |
| | 9.70 | 1H | H—20 |

(F)

| δ(ppm) | | |
|---|---|---|
| 1.82 | 3H | 22—Me |
| 2.27 | 6H | $NMe_2$ |
| 4.23 | 1H | H—1′ |
| 4.34 | 1H | H—23 |
| 4.91 | 1H | H—15 |
| 5.28~5.38 | 2H | H—25a,b |
| 5.79 | 1H | H—13 |
| 5.94 | 1H | H—24 |
| 6.31 | 1H | H—10 |
| 7.31 | 1H | H—11 |
| 9.71 | 1H | H—20 |

Example 11

(1)

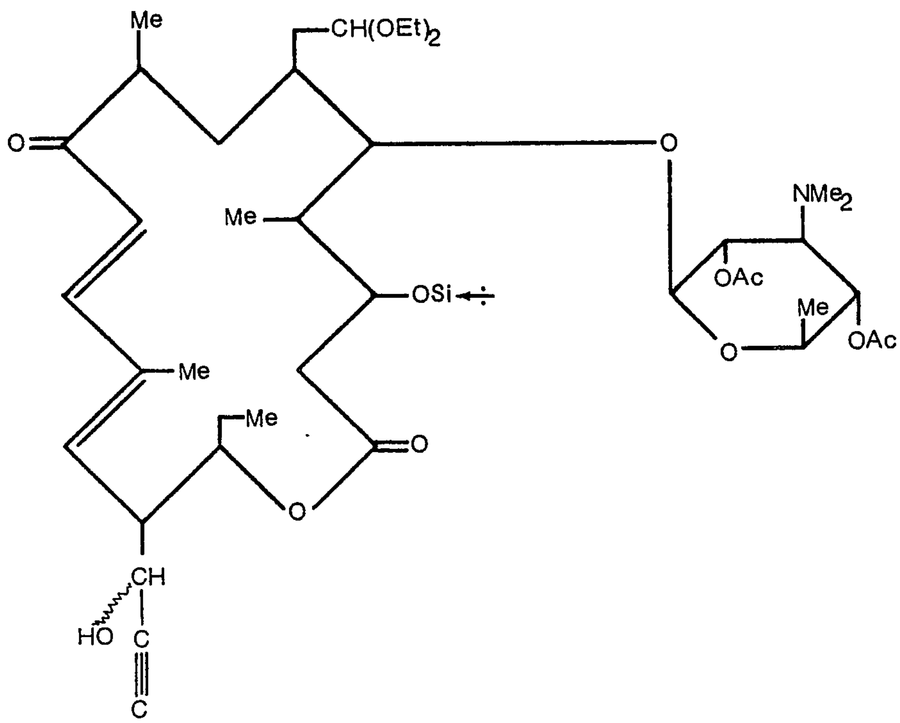

832 mg of 2′,4′-di-O-acetyl-3-O-tert-butyldimethylsilyl-23-deoxy-23-oxo-mycaminosyl tylonolide diethylacetal was treated with ethinyl magnesium bromide (tetrahydrofuran solution) to give 160 mg of one isomer (A) and 177 mg of another isomer (B) of 2′,4′-di-O-acetyl-3-O-tert-butyldimethylsilyl-23-C-ethynylmycaminosyl tylonolide diethylacetal as solid material.

NMR ($CDCl_3$)

(A)

| δ(ppm) | | |
|---|---|---|
| 0.90 | 9H | Si—tBu |
| 2.10 | 6H | 2′,4′—OCOMe |
| 2.41 | 6H | $NME_2$ |
| 6.08 | 1H | H—13 |
| 6.33 | 1H | H—10 |

(B)

| δ(ppm) | | |
|---|---|---|
| 0.90 | 9H | Si—tBu |
| 2.10 | 6H | 2′,4′—OCOMe |
| 2.40 | 6H | $NMe_2$ |
| 6.10 | 1H | H—13 |
| 6.30 | 1H | H—10 |

(2)

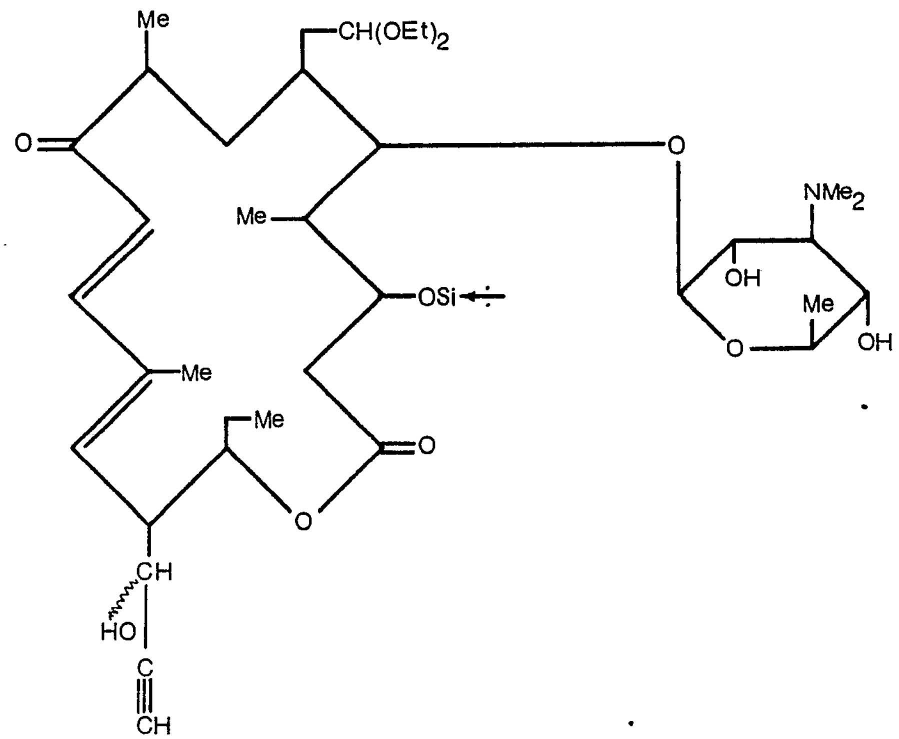

160 mg of the isomer (A) and 177 mg of the isomer (B) of 2′,4′-di-O-acetyl-3-O-tert-butyldimethylsilyl-23-C-ethylmycaminosyl tylonolide diethylacetal were treated as in Example 8 (2) to give respectively 109 mg of one isomer (C) and 129 mg of another isomer (D) of 3-O-tert-butyldimethylsilyl-23-C-ethynylmycaminosyl tylonolide diethylacetal as solid material.

NMR ($CDCl_3$)

| | | | |
|---|---|---|---|
| (C) | δ(ppm) | | |
| | 0.88 | 9H | Si—tBu |
| | 2.53 | 6H | $NMe_2$ |
| | 4.30 | 1H | H—1′ |
| | 6.05 | 1H | H—13 |
| | 6.32 | 1H | H—10 |
| | 7.30 | 1H | H—11 |
| (D) | δ(ppm) | | |
| | 0.88 | 9H | Si—tBU |
| | 2.55 | 6H | $NMe_2$ |
| | 4.33 | 1H | H—1′ |
| | 5.10 | 1H | H—15 |
| | 6.15 | 1H | H—13 |
| | 6.30 | 1H | H—10 |

(3)

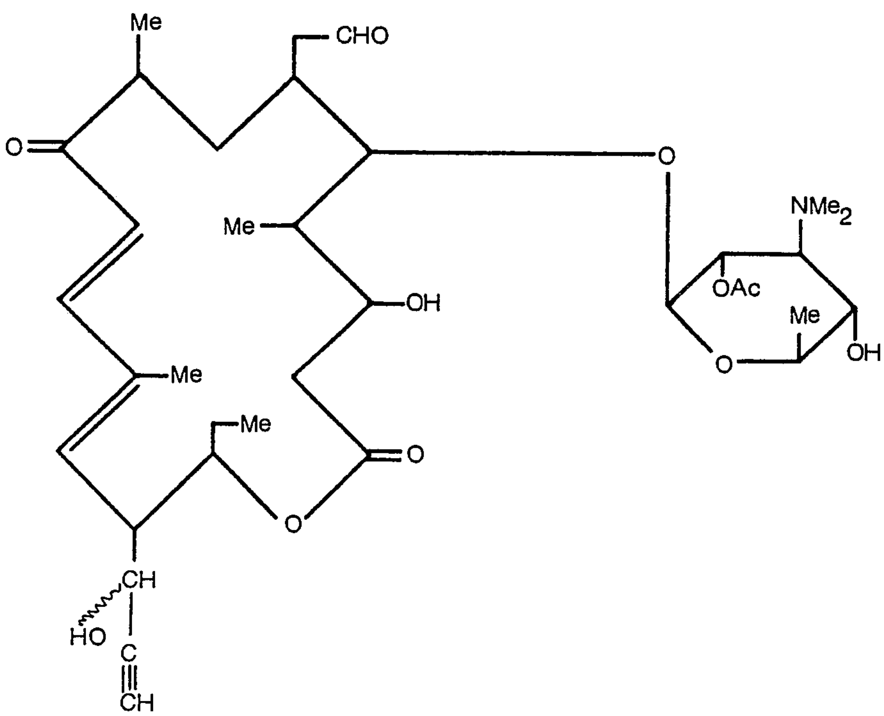

109 mg of the isomer (C) and 129 mg of the isomer (D) of 3-O-tert-butyldimethylsilyl-23-C-ethynylmycaminosyl tylonolide diethylacetal were treated as in Example 8 (3) to give respectively 75.5 mg of one isomer (E) or 101 mg of another isomer (F) of 23—C-ethynylmycaminosyl tylonolide as solid material.

NMR ($CDCl_2$)

(E) δ(ppm)

| | | |
|---|---|---|
| 1.84 | 3H | 22—Me |
| 2.51 | 6H | $NMe_2$ |
| 4.28 | 1H | H—1′ |
| 4.63 | 1H | H—23 |
| 5.23 | 1H | H—15 |
| 6.12 | 1H | H—13 |
| 6.34 | 1H | H—10 |
| 7.39 | 1H | H—11 |
| 9.70 | 1H | H—20 |

(F) δ(ppm)

| | | |
|---|---|---|
| 1.85 | 3H | 22—Me |
| 2.51 | 6H | $NMe_2$ |
| 2.74 | 1H | H—25 |
| 4.27 | 1H | H—1′ |
| 4.58 | 1H | H—23 |
| 5.10 | 1H | H—15 |
| 6.12 | 1H | H—13 |
| 6.30 | 1H | H—10 |
| 7.43 | 1H | H—11 |
| 9.69 | 2H | H—20 |

Example 12

(1)

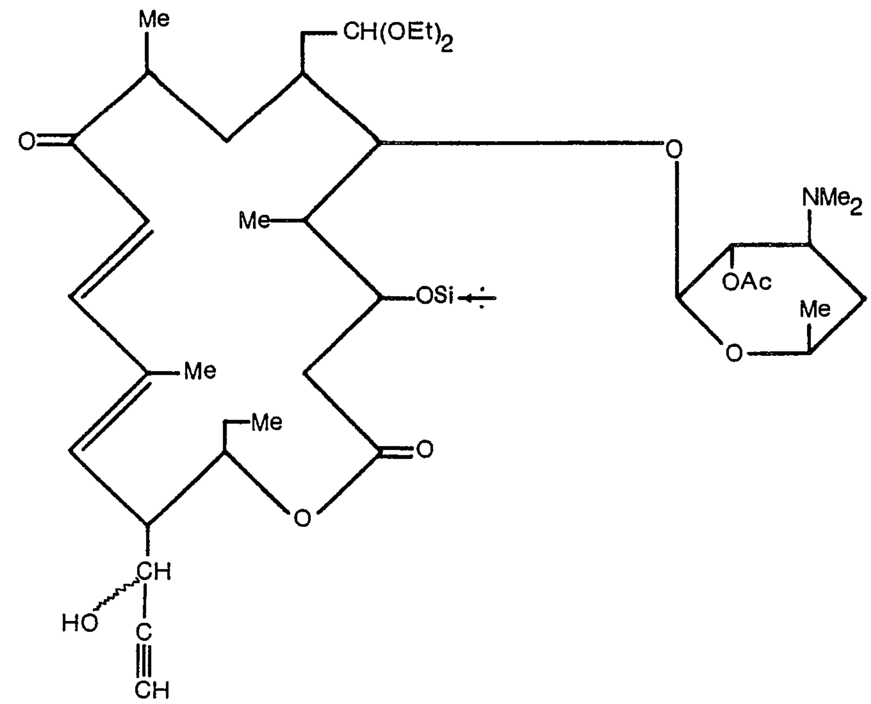

814 mg of 2'-O-acetyl-3-O-tert-butyldimethylsilyl-4'-deoxymycaminosyltylonolide diethylacetal was treated as in Example 11 (1) to give 103 mg of one isomer (A) and 118 mg of another isomer (B) of 2'-O-acetyl-3-O-tert-butyldimethyl-4'-deoxy-23-C-ethynylmycaminosyl tylonolide diethylacetal as solid material.

NMR ($CDCl_3$)

| | | | |
|---|---|---|---|
| (A) | δ(ppm) | | |
| | 0.90 | 9H | Si—tBu |
| | 2.12 | 3H | 2'—OCOMe |
| | 2.30 | 6H | $NMe_2$ |
| | 6.07 | 1H | H—13 |
| | 6.33 | 1H | H—10 |
| (B) | δ(ppm) | | |
| | 0.89 | 9H | Si—tBu |
| | 2.10 | 3H | 2'—OCCMe |
| | 2.30 | 6H | $NMe_2$ |
| | 6.03 | 1H | H—13 |
| | 6.30 | 1H | H—10 |

(2)

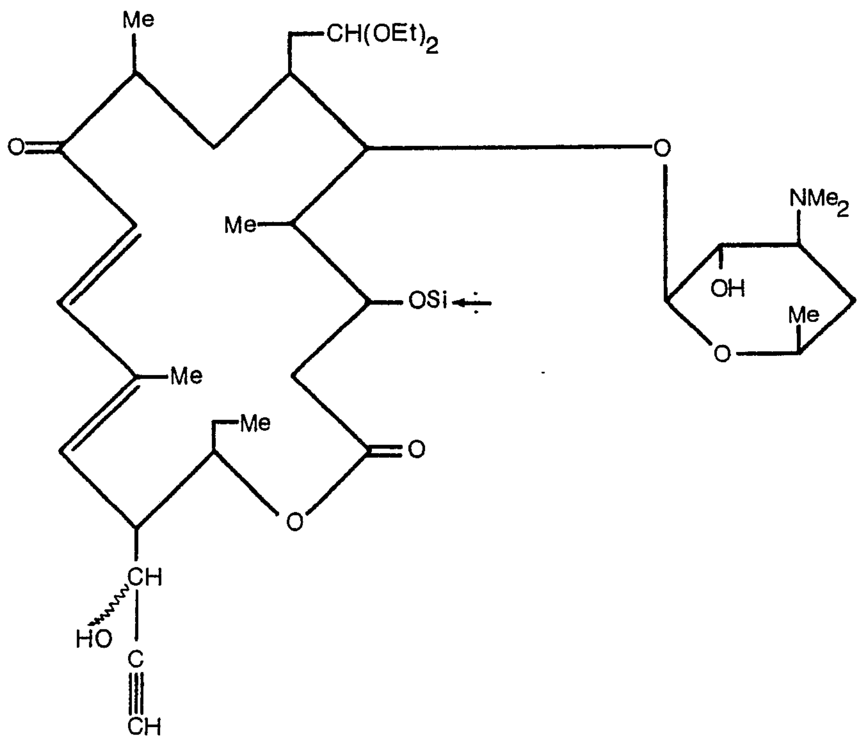

103 mg of the isomer (A) and 118 mg of the isomer (B) of 2'-O-acetyl-3-O-tert-butyldimethylsilyl-4'-deoxy-23-C-ethynylmycaminosyl tylonolide diethylacetal were treated as in Example 8 (2) to give respectively 62 mg of one isomer (C) or 70 mg of another isomer (D) of 3-O-tert-butyldimethylsilyl-4'-deoxy-23-C-ethynylmycaminosyl tylonolide diethylacetal as solid material.

NMR ($CDCl_3$)

| | | | |
|---|---|---|---|
| (C) | δ(ppm) | | |
| | 0.88 | 9H | Si—tBu |
| | 2.31 | 6H | $NMe_2$ |
| | 5.28 | 1H | H—15 |
| | 6.07 | 1H | H—13 |
| | 6.33 | 1H | H—10 |
| (D) | δ(ppm) | | |
| | 0.87 | 9H | Si—tBu |
| | 2.30 | 6H | $NMe_2$ |
| | 5.03 | 1H | H—15 |
| | 6.07 | 1H | H—13 |
| | 6.30 | 1H | H—10 |

(3)

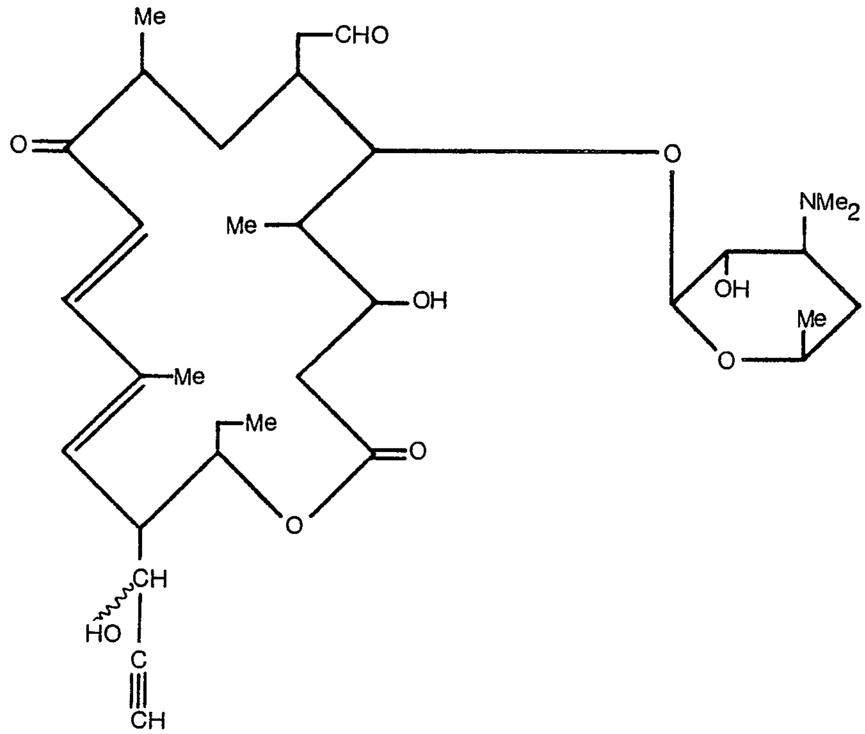

62 mg of the isomer (C) and 70 mg of the isomer (D) of 3-O-tert-butyldimethylsilyl-4'-deoxy-23-C-ethynylmycaminosyl tylonolide diethylacetal were treated as in Example 8 (3) to give respectively 38 mg of one isomer (E) and 40 mg of another isomer (F) of 4'-deoxy-23-C-ethynylmycaminosyl tylonolide as solid material.

NMR ($CDCl_3$)

| | | | |
|---|---|---|---|
| (E) | δ(ppm) | | |
| | 1.82 | 3H | 22—Me |
| | 2.27 | 6H | $NMe_2$ |
| | 2.50 | 1H | H—25 |
| | 4.23 | 1H | H—1' |
| | 4.64 | 1H | H—23 |
| | 5.23 | 1H | H—15 |
| | 6.13 | 1H | H—13 |
| | 6.34 | 1H | H—10 |
| | 7.42 | 1H | H—11 |
| | 9.72 | 1H | H—20 |

(F)

| δ(ppm) | | |
|---|---|---|
| 1.84 | 3H | 22—$Me_2$ |
| 2.28 | 6H | $NMe_2$ |
| 2.70 | 1H | H—25 |
| 4.22 | 1H | H—1′ |
| 4.57 | 1H | H—23 |
| 5.08 | 1H | H—15 |
| 6.11 | 1H | H—13 |
| 6.35 | 1H | H—10 |
| 7.43 | 1H | H—11 |
| 9.72 | 1H | H—20 |

**Claims**

1. A 23-C-substituted mycaminosyl tylonolide compound of the following general formula (I):

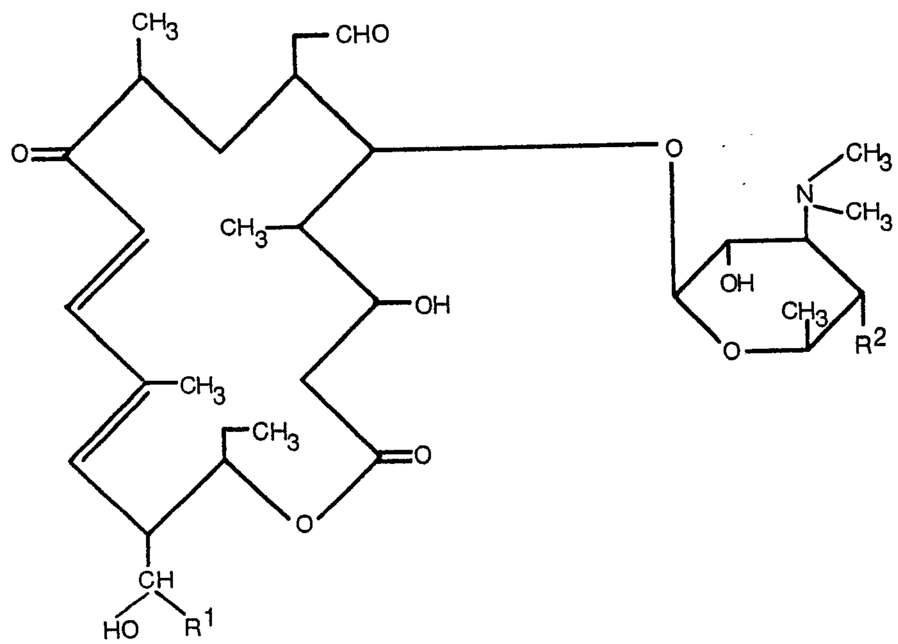

(I)

wherein $R^1$ represents a straight or branched $C_1$ to $C_5$ alkyl group, an aryl group, a straight or branched $C_2$ to $C_6$ alkenyl group or a straight or branch $C_2$ to $C_6$ alkynyl group, and $R^2$ represents a hydrogen atom or a hydroxyl group.

2. A compound according to claim 1 wherein $R^1$ represents a staight or branched $C_1$ to $C_5$ alkyl group, a staight or branched $C_2$ to $C_6$ alkenyl group, or a straight or branched $C_2$ to $C_6$ alkynyl group.

3. A process for producing a compound according to claim 1 which is characterized by reacting a compound of the formula:

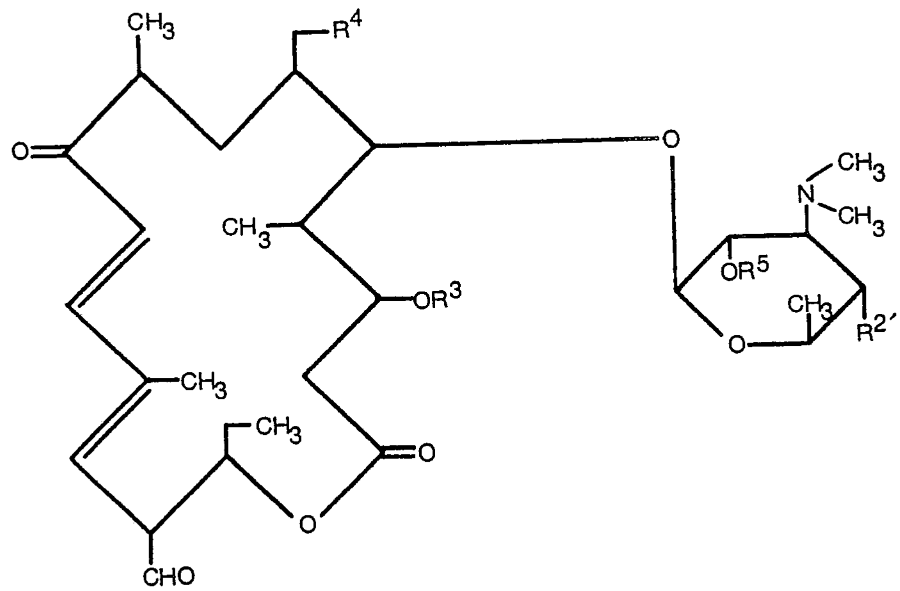

(II)

with Grignard reagent of the formula $R^1MgX$ or with organic lithium compound of the formula $R^1$—Li and then releasing the hydroxyl- and aldehyde-protective groups wherein $R^{2'}$ represents a hydrogen atom or a protected hydroxyl group, $R^3$ represents a protective group for a hydroxyl group, $R^4$ represents a protected aldehyde group, $R^5$ represents a protective group for a hydroxyl group; $R^1$ and $R^2$ are as defined in claim 1 and X represents a halogen atom.

4. An antibacterial composition containing as an effective component at least one compound according to claim 1.

**Patentansprüche**

1. 23-C-substituierte Mykaminosyltylonolidverbindung der nach- stehenden allgemeinen Formel (I):

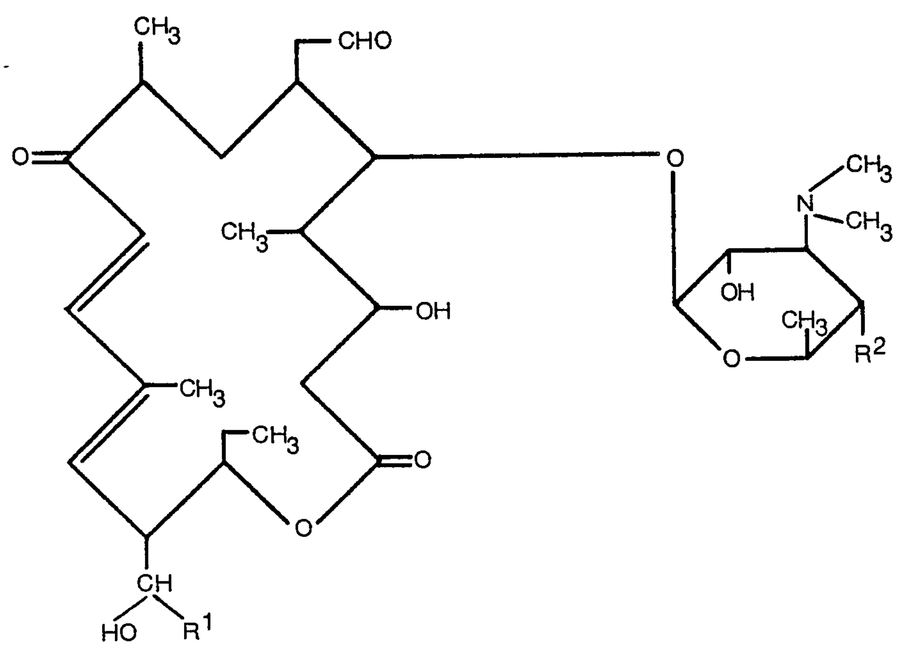

(I)

worin $R^1$ eine geradkettige oder verzweigte $C_1$- bis $C_5$-Alkylgruppe, eine Arylgruppe, eine geradkettige oder verzweigte $C_2$- bis $C_6$-Alkenylgruppe oder eine geradkettige oder verzweigte $C_2$- bis $C_6$-Alkenylgruppe darstellt und $R^2$ ein Wasserstoffatom oder eine Hydroxylgruppe darstellt.

2. Verbindung nach Anspruch 1, worin $R^1$ eine geradkettige oder verzweigte $C_1$- bis $C_5$-Alkylgruppe, eine geradkettige oder verzweigte $C_2$- bis $C_6$-Alkenylgruppe oder eine geradkettige oder verzweigte $C_2$- bis $C_6$-Alkenylgruppe darstellt.

3. Verfahren zur Herstellung einer Verbindung nach Anspruch 1, dadurch gekennzeichnet, daß eine Verbindung der Formel

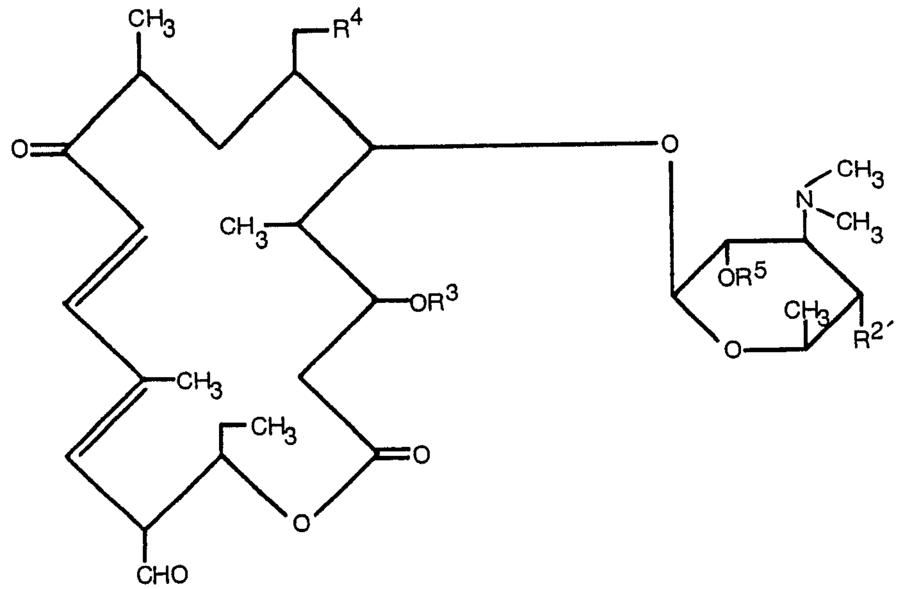

(II)

mit einem Grigardreagens der Formel $R^1MgX$ oder mit einer organischen Lithiumverbindung der Formel $R^1$—Li umgesetzt wird und dann die Hydroxyl- und Aldehyd-Schutzgruppen freigesetzt werden, wobei $R^{2'}$ ein Wasserstoffatom oder eine geschützte Hydroxylgruppe darstellt, $R^3$ eine Schutzgruppe für eine Hydroxylgruppe darstellt, $R^4$ eine geschützte Aldehydgruppe darstellt, $R^5$ eine Schutzgruppe für eine Hydroxylgruppe darstellt; $R^1$ und $R^2$ wie in Anspruch 1 definiert sind und X ein Halogenatom darstellt.

4. Antibakterielle Zusammensetzung, enthaltend als wirksamen Bestandteil wenigstens eine Verbindung nach Anspruch 1.

**Revendications**

1. Un composé de mycaminosyl-tylonolide substitué par C en position 23 de formule générale (I) suivante:

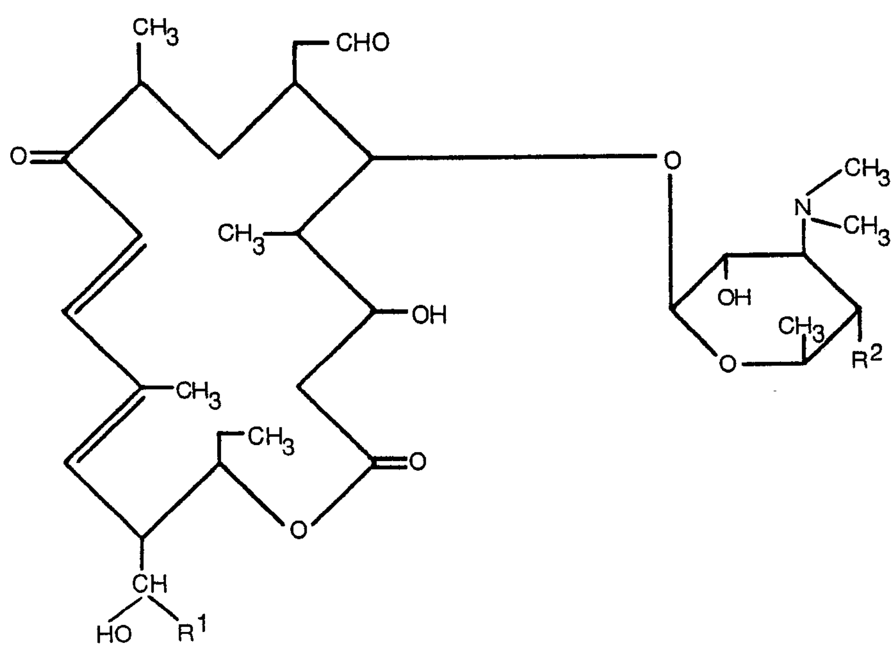

(I)

dans laquelle $R^1$ représente un groupe alkyle droit ou à chaîne ramifiée en $C_1$ à $C_5$, un groupe aryle, un groupe alkényle droit ou à chaîne ramifiée en $C_2$ à $C_6$ ou un groupe alkynyle droit ou à chaîne ramifiée en $C_2$ à $C_6$, et $R^2$ représente un atome d'hydrogène ou un groupe hydroxyle.

2. Un composé selon la revendication 1, dans lequel $R^1$ représente un groupe alkyle droit ou a chaîne ramifiée en $C_1$ à $C_5$, un groupe alkényle droit ou à chaîne ramifiée en $C_2$ à $C_6$, ou un groupe alkynyle droit ou à chaîne ramifiée en $C_2$ à $C_6$.

3. Un procédé de fabrication d'un composé selon la revendication 1, qui est caractérisé en ce qu'on fait réagir un composé de formule:

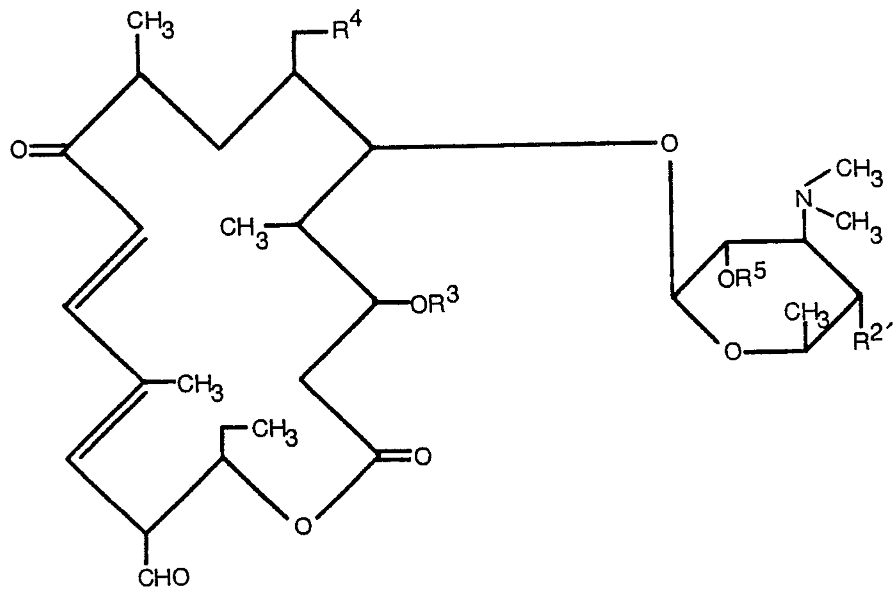

(II)

avec un réactif de Grignard de formule $R^1MgX$ ou avec un composé organique de lithium de formule $R^1$—Li et on libère ensuite les groupes protecteurs hydroxyle et aldéhyde, dans laquelle $R^{2'}$ représente un atome d'hydrogène ou un groupe hydroxyle protégé, $R^3$ représente un groupe protecteur pour un groupe hydroxyle, $R^4$ représente un groupe aldéhyde protégé, $R^5$ représente un groupe protecteur pour un groupe hydroxyle; $R^1$ et $R^2$ sont définis comme à la revendication 1 et X représente un atome d'halogène.

4. Une composition antibactérienne contenant comme composant efficace au moins un composé selon la revendication 1.